# FASCICULE DE BREVET EUROPEEN

(11) **EP 1 720 829 B1**
(45) Date de publication et mention de la délivrance du brevet: **14.10.2009**
(21) Numéro de dépôt: 05732902.1
(22) Date de dépôt: 25.02.2005
(51) Int. Cl.: C07C 271/10, C07D 215/04, C07D 237/08, C07D 233/54, C07D 235/14, C07D 263/32, C07D 471/04

(54) **DERIVES D' HETEROARYL-ALKYLCARBAMATES, LEUR PREPARATION ET LEUR APPLICATION COMME INHIBITEURS DE L'ENZYME FAAH**
DERIVATE VON HETEROARYLALKYLCARBAMATEN, VERFAHREN ZU DEREN HERSTELLUNG UND DEREN VERWENDUNG ALS FAAH-ENZYMINHIBITOREN
DERIVATIVES OF HETEROARYL-ALKYLCARBAMATES, PREPARATION METHOD THEREOF AND USE OF SAME AS FAAH ENZYME INHIBITORS

(30) Priorité: 26.02.2004 FR 0401949
(43) Date de publication de la demande: 15.11.2006
(73) Titulaire: Sanofi-Aventis, 75013 Paris (FR)
(72) Inventeur: ABOUABDELLAH, Ahmed, F-94320 Thiais (FR); BARTSCH-LI, Régine, F-92260 Fontenay-aux-Roses (FR); HOORNAERT, Christian, F-92160 Antony (FR); RAVET, Antoine, F-92500 Rueil-Malmaisoin (FR)
(74) Mandataire: Morel-Pécheux, Muriel
(86) Numéro de dépôt international: PCT/FR2005/000451
(87) Numéro de publication internationale: WO 2005/090292

(56) Documents cités:
- WO-A-99/26584
- WO-A-02/087569
- WO-A-20/04067498
- WO-A2-20/04020430
- US-A- 2 866 734
- US-A- 3 054 794
- US-A- 3 876 661
- TARZIA G ET AL: "Design, synthesis, and structure-activity relationships of alkylcarbamic acid aryl esters, a new class of Fatty Acid Amide Hydrolase inhibitors" JOURNAL OF MEDICINAL AND PHARMACEUTICAL CHEMISTRY, AMERICAN CHEMICAL SOCIETY. EASTON, US, vol. 46, no. 12, 2003, pages 2352-2360, XP002257137
- GRAZIA CASCIO M ET AL: "A structure-activity relationship study on N-arachidonoyl-amino acids as possible endogenous inhibitors of fatty acid amide hydrolase" BIOCHEMICAL AND BIOPHYSICAL RESEARCH COMMUNICATIONS, ACADEMIC PRESS INC. ORLANDO, FL, US, vol. 314, no. 1, 30 janvier 2004 (2004-01-30), pages 192-196, XP004483580 ISSN: 0006-291X
- SHAPIRO S L ET AL: "Aminoalkylamides and Oxazolidinediones" JOURNAL OF THE AMERICAN CHEMICAL SOCIETY, AMERICAN CHEMICAL SOCIETY, WASHINGTON, DC, US, vol. 81, no. 12, 1959, pages 3083-3088, XP002285705 ISSN: 0002-7863

## Description

L'invention a pour objet des dérivés d'aryl- et d'hétéroaryl-alkylcarbamates, leur préparation et leur application en thérapeutique.

On connaît déjà des dérivés de phénylalkylcarbamates et de dioxane-2-alkylcarbamates, décrits respectivement dans les documents FR2850377 A et WO2004/020430 A2, inhibiteurs de l'enzyme FAAH (Fatty Acid Amido Hydrolase). WO02/087569 décrit des dérivés de *(*4,5-diphényl-imidazolyl)alkyl-carbamates comme inhibiteurs de l'enzyme FAAH.
Il existe toujours une nécessité de trouver et de développer des produits inhibiteurs de l'enzyme FAAH. Les composés de l'invention répondent à ce but.

Les composés de l'invention répondent à la formule générale (I) : 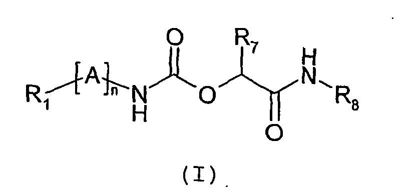 dans laquelle
A est choisi parmi un ou plusieurs groupes X, Y et/ou Z ;
X représente un groupe méthylène éventuellement substitué par un ou deux groupes C₁₋₆-alkyle, C₃₋₇-cycloalkyle ou C₃₋₇-cycloalkyl-C₁₋₃-alkylène ;
Y représente soit un groupe C₂-alcènylène éventuellement substitué par un ou deux groupes C₁₋₆-alkyle, C₃₋₇-cycloalkyle ou C₃₋₇-cycloalkyl-C₁₋₃-alkylène ; soit un groupe C₂-alcynylène ;
Z représente un groupe de formule : 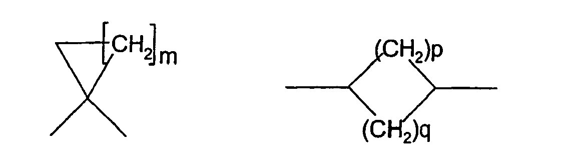 m représente un nombre entier allant de 1 à 5 ;
p et q représentent des nombres entiers et sont définis tels que p+q soit un nombre allant de 1 à 5 ;
n représente un nombre entier allant de 1 à 7 ;
R₁ représente un groupe R₂ éventuellement substitué par un ou plusieurs groupes R₃ et/ou R₄ ;
R₂ représente un groupe choisi parmi un pyridinyle, pyrimidinyle, pyridazinyle, pyrazinyle, triazinyle, pyrrolyle, furanyle, thiènyle, imidazolyle, oxazolyle, thiazolyle, pyrazolyle, isoxazolyle, isothiazolyle, oxadiazolyle, thiadiazolyle, triazolyle, tétrazolyle, naphtalènyle, quinolinyle, tétrahydroquinolinyle, isoquinolinyle, tétrahydroisoquinolinyle, 2-oxo-3,4-dihydroquinolinyle, 1-oxo-3,4-dihydroisoquinolinyle, quinazolinyle, quinoxalinyle, phthalazinyle, cinnolinyle, naphthyridinyle, benzofuranyle, dihydrobenzofuranyle, benzothiènyle, dihydrobenzothiènyle, indolyle, indolinyle, indazolyle, isoindolyle, benzimidazolyle, benzoxazolyle, benzisoxazolyle, benzothiazolyle, benzisothiazolyle, benzotriazolyle, benzoxadiazolyle, benzothiadiazolyle, pyrrolopyridinyle, furopyridinyle, thiènopyridinyle, imidazopyridinyle, oxazolopyridinyle, thiazolopyridinyle, pyrazolopyridinyle, isoxazolopyridinyle, isothiazolopyridinyle ;
R₃ représente un groupe choisi parmi les atomes d'halogènes, les groupes cyano, nitro, C₁₋₆-alkyle, C₁₋₆-alcoxy, hydroxyle, C₁₋₆-thioalkyle, C₁₋₆-fluoroalkyle, C₁₋₆-fluoroalcoxy, C₁₋₆-fluorothioalkyle, NR₅R₆, NR₅COR₆, NR₅CO₂R₆, NR₅SO₂R₆, COR₅, CO₂R₅, CONR₅R₆, SO₂R₅, SO₂NR₅R₆, -O- (C₁₋₃-alkylène) -O ;
R₄ représente un groupe choisi parmi les groupes phényle, phényloxy, benzyloxy, naphtalènyle, pyridinyle, pyrimidinyle, pyridazinyle, pyrazinyle ; le ou les groupes R₄ pouvant être substitués par un ou plusieurs groupes R₃ identiques ou différents l'un de l'autre ;
R₅ et R₆ représentent indépendamment l'un de l'autre un atome d'hydrogène ou un groupe C₁₋₆-alkyle, ou forment avec le ou les atomes qui les portent un cycle choisi parmi un cycle azétidine, pyrrolidine, pipéridine, morpholine, thiomorpholine, azépine ou pipérazine, ce cycle étant éventuellement substitué par un groupe C₁₋₆-alkyle ou benzyle ;
R₇ représente un atome d'hydrogène ou un groupe C₁₋₆-alkyle ;
R₈ représente un atome d'hydrogène ou un groupe C₁₋₆-alkyle, C₃₋₇-cycloalkyle, C₃₋₇-cycloalkyle-C₁₋₃-alkylène .

Dans le cadre de l'invention, les composés de formule générale (I) peuvent donc comporter plusieurs groupes A identiques ou différents entre eux.

Parmi les composés de formule générale (I), un premier sous-groupe de composés est constitué des composés pour lesquels :
A est choisi parmi un ou plusieurs groupes X et/ou Y ;
X représente un groupe méthylène;
Y représente un groupe C₂-alcynylène ;
n représente un nombre entier allant de 1 à 5 ;
R₁ représente un groupe R₂ éventuellement substitué par un ou plusieurs groupes R₃ et/ou R₄ ;
R₂ représente un groupe choisi parmi un pyridinyle, pyrimidinyle, pyridazinyle, imidazolyle, oxazolyle, pyrazolyle, isoxazolyle, oxadiazolyle, naphtalènyle, quinolinyle, isoquinolinyle, dihydroisoquinolinyle, 2-oxo-3,4-dihydroquinolinyle, indolyle, benzimidazolyle, pyrrolopyridinyle ;
R₃ représente un groupe choisi parmi les atomes d'halogènes, plus particulièrement les atomes de chlore et de fluor, les groupes C₁₋₆-alkyle, plus particulièrement méthyle, C₁₋₆-alcoxy, plus particulièrement méthoxy, NR₅R₆;
R₄ représente un groupe choisi parmi les groupes phényle, naphtalènyle, pyridinyle ; le ou les groupes R₄ pouvant être substitués par un ou plusieurs groupes R₃ identiques ou différents l'un de l'autre ;
R₅ et R₆ représentent indépendamment l'un de l'autre un groupe C₁₋₆-alkyle, plus particulièrement un méthyle ;
R₇ représente un atome d'hydrogène ou un groupe C₁₋₆-alkyle ; R₈ représente un atome d'hydrogène ou un groupe C₁₋₆-alkyle, C₃₋₇-cycloalkyle, C₃₋₇-cycloalkyle-C₁₋₃-alkylène.

Parmi les composés de formule générale (I), un second sous-groupe de composés est constitué des composés pour lesquels :
A, X, Y, Z, m, p, q, n, R₁, R₃, R₄, R₅, R₆, R₇, R₈ sont tels que définis dans la formule générale (I) ou dans le premier sous-groupe tel que défini ci-dessus,
et R₂ représente un groupe choisi parmi un pyridinyle, pyrimidinyle, oxazolyle, isoxazolyle, naphtalènyle, quinolinyle, isoquinolinyle.

Parmi les composés de formule générale (I), un troisième sous-groupe de composés est constitué des composés pour lesquels :
A, X, Y, Z, m, p, q, n, R₁, R₂, R₃, R₄, R₅, R₆ sont tels que définis dans la formule générale (I) ou dans les sous-groupes tels que définis ci-dessus ;
R₇ représente un atome d'hydrogène
R₈ représente un atome d'hydrogène ou un groupe C₁₋₆-alkyle, plus particulièrement un méthyle.

Parmi les composés des sous-groupes définis ci-dessus, les composés suivants peuvent être cités :
- [(5-phénylpyridin-2-yl)méthyl]carbamate de 2-(méthylamino) -2-oxoéthyle
- 2-(5-phénylpyridin-2-yl)éthylcarbamate de 2-(méthylamino)-2-oxoéthyle
- 2-(6-phénylpyridin-3-yl)éthylcarbamate de 2-(méthylamino)-2-oxoéthyle
- 2-(2-phénylpyrimidin-5-yl)éthylcarbamate de 2-(méthylamino)-2-oxoéthyle
- 2-(5-phénylpyrimidin-2-yl)éthylcarbamate de 2-(méthylamino)-2-oxoéthyle
- 2-[6-(4-chlorophényl)-pyrimidin-4-yl]éthylcarbamate de 2-(méthylamino)-2-oxoéthyle
- 2-[6-(4-chlorophényl)-2-méthyl-pyrimidin-4-yl]éthylcarbamate de 2-(méthylamino)-2-oxoéthyle
- 2-[6-(4-chlorophényl)-2-(diméthylamino)-pyrimidin-4-yl]éthylcarbamate de 2-(méthylamino)-2-oxoéthyle
- (2-isoquinolin-7-yléthyl)carbamate de 2-(méthylamino)-2-oxoéthyle
- 2-(2-phényl-1,3-oxazol-4-yl)éthylcarbamate de 2-(méthylamino)-2-oxoéthyle
- 2-[5-(4-chlorophényl)-isoxazol-3-ylléthylcarbamate de 2-(méthylamino)-2-oxoéthyle
- (3-pyridin-2-ylpropyl)carbamate de 2-amino-2-oxoéthyle
- (3-pyridin-3-ylpropyl)carbamate de 2-amino-2-oxoéthyle
- (3-pyridin-4-ylpropyl)carbamate de 2-amino-2-oxoéthyle
- (3-pyrimidin-5-ylpropyl)carbamate de 2-amino-2-oxoéthyle
- 3-[6-(4-chlorophényl)-pyrimidin-4-yl]propylcarbamate de 2 - (méthylamino)-2-oxoéthyle
- 3-[6-(4-chlorophényl)-2-méthyl-pyrimidin-4-yl]propylcarbamate de 2-(méthylamino)-2-oxoéthyle
- 3-[6-(4-chlorophényl)-2-(diméthylamino)-pyrimidin-4-yl]propylcarbamate de 2-(méthylamino)-2-oxoéthyle
- (3-quinolin-2-ylpropyl)carbamate de 2-amino-2-oxoéthyle
- (3-quinolin-4-ylpropyl)carbamate de 2-amino-2-oxoéthyle
- (3-isoquinolin-1-ylpropyl)carbamate de 2-amino-2-oxoéthyle
- (3-isoquinolin-4-ylpropyl)carbamate de 2-amino-2-oxoéthyle
- 3-[5-(4-chlorophényl)-isoxazol-3-yl]propylcarbamate de 2-(méthylamino)-2-oxoéthyle
- 3-[3-(4-chlorophényl)-isoxazol-5-yl]propylcarbamate de 2-amino-2-oxoéthyle,
- 3-[3-(4-chlorophényl)-isoxazol-5-yl]propylcarbamate de 2-(méthylamino)-2-oxoéthyle
- 3-[3-(4-phényl-phényl)-isoxazol-5-yl)propylcarbamate de 2-(méthylamino)-2-oxoéthyle
- 3-[3-(naphtalèn-2-yl)-isoxazol-5-yl]propylcarbamate de 2-(méthylamino)-2-oxoéthyle
- (4-pyridin-2-ylbutyl)carbamate de 2-amino-2-oxoéthyle
- (4-pyridin-3-ylbutyl)carbamate de 2-amino-2-oxoéthyle
- (4-pyridin-4-ylbutyl)carbamate de 2-amino-2-oxoéthyle
- (4-pyrimidin-5-ylbutyl)carbamate de 2-amino-2-oxoéthyle
- 4-[6-(4-chlorophényl)-pyrimidin-4-yl]butylcarbamate de 2-(méthylamino)-2-oxoéthyle
- 4-[6-(4-chlorophényl)-2-méthyl-pyrimidin-4-yl]butylcarbamate de 2-(méthylamino)-2-oxoéthyle
- 4-[6-(4-chlorophényl)-2-cyclopropyl-pyrimidin-4-yl]butylcarbamate de 2-(méthylamino)-2-oxoéthyle
- 4-[6-(4-chlorophényl)-2-(diméthylamino)-pyrimidin-4-yl]butylcarbamate de 2-(méthylamino)-2-oxoéthyle
- (4-quinolin-2-ylbutyl)carbamate de 2-amino-2-oxoéthyle
- (4-quinolin-4-ylbutyl)carbamate de 2-amino-2-oxoéthyle
- (4-isoquinolin-1-ylbutyl)carbamate de 2-amino-2-oxoéthyle
- (4-isoquinolin-4-ylbutyl)carbamate de 2-amino-2-oxoéthyle
- 4-[5-(4-chlorophényl)-isoxazol-3-yl]butylcarbamate de 2-(méthylamino)-2-oxoéthyle
- 4-[3-(4-chlorophényl)-isoxazol-5-yl]butylcarbamate de 2-(méthylamino)-2-oxoéthyle
- 4-[3-(4-phényl-phényl)-isoxazol-5-yl]butylcarbamate de 2-(méthylamino)-2-oxoéthyle
- 4-[3-(naphtalèn-2-yl)-isoxazol-5-yl]butylcarbamate de 2-(méthylamino)-2-oxoéthyle
- (5-pyridin-2-ylpentyl)carbamate de 2-amino-2-oxoéthyle
- (5-pyridin-4-ylpentyl)carbamate de 2-amino-2-oxoéthyle
- (5-pyrimidin-5-ylpentyl)carbamate de 2-amino-2-oxoéthyle
- (5-quinolin-2-ylpentyl)carbamate de 2-amino-2-oxoéthyle
- (5-quinolin-4-ylpentyl)carbamate de 2-amino-2-oxoéthyle
- (5-isoquinolin-1-ylpentyl)carbamate de 2-amino-2-oxoéthyle
- (5-isoquinolin-4-ylpentyl)carbamate de 2-amino-2-oxoéthyle
- (3-naphthalèn-1-ylprop-2-yn-1-yl)carbamate de 2-amino-2-oxoéthyle
- (3-naphthalèn-1-ylprop-2-yn-1-yl)carbamate de 2-(méthylamino)-2-oxoéthyle
- (5-naphthalèn-1-ylpent-4-yn-1-yl)carbamate de 2-amino-2-oxoéthyle
- (5-naphthalèn-1-ylpent-4-yn-1-yl)carbamate de 2-(méthylamino)-2-oxoéthyle
- [5-(4-fluoro-naphthalèn-1-yl)pent-4-yn-1-yl]carbamate de 2-(méthylamino)-2-oxoéthyle
- [3-(pyridin-3-yl)-isoxazol-5-ylpropyl]carbamate de 2-(méthylamino)-2-oxoéthyle
- [3-(4-méthoxynaphtalèn-l-yl)-isoxazol-5-ylpropyl]carbamate de 2-(méthylamino)-2-oxoéthyle.

Parmi les composés de formule générale (I), une sous-famille de composés est constituée des composés répondant à la formule générale (I') : 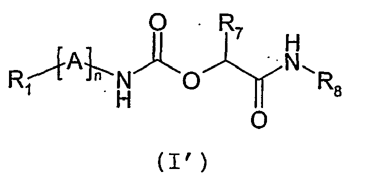 dans laquelle
A est choisi parmi un ou plusieurs groupes X, Y et/ou Z ;
X représente un groupe méthylène éventuellement substitué par un ou deux groupes C₁₋₆-alkyle, C₃₋₇-cycloalkyle ou C₃₋₇-cycloalkyl-C₁₋₃-alkylène ;
Y représente soit un groupe C₂-alcènylène éventuellement substitué par un ou deux groupes C₁₋₆-alkyle, C₃₋₇-cycloalkyle ou C₃₋₇-cycloalkyl-C₁₋₃-alkylène ; soit un groupe
C₂-alcynylène ;
Z représente un groupe de formule : 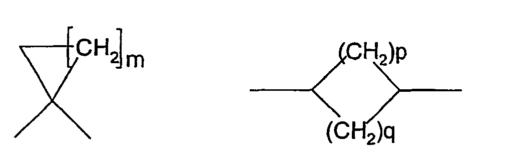 m représente un nombre entier allant de 1 à 5 ;
p et q représentent des nombres entiers et sont définis tels que p+q soit un nombre allant de 1 à 5 ;
n représente un nombre entier allant de 1 à 7 ;
R₁ représente un groupe R₂ éventuellement substitué par un ou plusieurs groupes R₃ et/ou R₄ ;
R₂ représente un groupe choisi parmi un pyridinyle, pyrimidinyle, pyridazinyle, pyrazinyle, triazinyle, pyrrolyle, furanyle, thiènyle, imidazolyle, oxazolyle, thiazolyle, pyrazolyle, isoxazolyle, isothiazolyle, oxadiazolyle, thiadiazolyle, triazolyle, tétrazolyle, naphtalènyle, quinolinyle, tétrahydroquinolinyle, isoquinolinyle, tétrahydroisoquinolinyle, 2-oxo-3,4-dihydroquinolinyle, 1-oxo-3,4-dihydroisoquinolinyle, quinazolinyle, quinoxalinyle, phthalazinyle, cinnolinyle, naphthyridinyle, benzofuranyle, dihydrobenzofuranyle, benzothiènyle, dihydrobenzothiènyle, indolyle, indolinyle, indazolyle, isoindolyle, benzimidazolyle, benzoxazolyle, benzisoxazolyle, benzothiazolyle, benzisothiazolyle, benzotriazolyle, benzoxadiazolyle, benzothiadiazolyle, pyrrolopyridinyle, furopyridinyle, thiènopyridinyle, imidazopyridinyle, oxazolopyridinyle, thiazolopyridinyle, pyrazolopyridinyle, isoxazolopyridinyle, isothiazolopyridinyle ;
R₃ représente un groupe choisi parmi les atomes d'halogènes ou les groupes cyano, nitro, C₁₋₆-alkyle, C₁₋₆-alcoxy, hydroxyle, C₁₋₆-thioalkyle, C₁₋₆-fluoroalkyle, C₁₋₆-fluoroalcoxy, C₁₋₆-fluorothioalkyle, NR₅R₆, NR₅COR₆, NR₅CO₂R₆, NR₅SO₂R₆, COR₅, CO₂R₅, CONR₅R₆, SO₂R₅, SO₂NR₅R₆, -O- (C₁₋₃-alkylène) -O ;
R₄ représente un groupe choisi parmi les groupes phényle, phényloxy, benzyloxy, naphtalènyle, pyridinyle, pyrimidinyle, pyridazinyle, pyrazinyle ; le ou les groupes R₄ pouvant être substitués par un ou plusieurs groupes R₃ identiques ou différents l'un de l'autre ;
R₅ et R₆ représentent indépendamment l'un de l'autre un atome d'hydrogène ou un groupe C₁₋₆-alkyle, ou forment avec le ou les atomes qui les portent un cycle choisi parmi un cycle azétidine, pyrrolidine, pipéridine, morpholine, thiomorpholine, azépine ou pipérazine, ce cycle étant éventuellement substitué par un groupe C₁₋₆-alkyle ou benzyle ;
R₇ représente un atome d'hydrogène ou un groupe C₁₋₆-alkyle ;
R₈ représente un atome d'hydrogène ou un groupe C₁₋₆-alkyle, C₃₋₇-cycloalkyle, C₃₋₇-cycloalkyle-C₁₋₃-alkylène.

Parmi les composés de formule générale (I'), un premier sous-groupe de composés est constitué des composés pour lesquels :
A est choisi parmi un ou plusieurs groupes X et/ou Y ;
X représente un groupe méthylène;
Y représente un groupe C₂-alcynylène
n représente un nombre entier allant de 1 à 5 ;
R₁ représente un groupe R₂ éventuellement substitué par un ou plusieurs groupes phényle, plus particulièrement par un phényle ;
R₂ représente un groupe choisi parmi un pyridinyle, pyrimidinyle, pyridazinyle, imidazolyle, oxazolyle, oxadiazolyle, naphtalènyle, quinolinyle, isoquinolinyle, dihydroisoquinolinyle, 2-oxo-3,4-dihydroquinolinyle, indolyle, benzimidazolyle, pyrrolopyridinyle ;
R₇ représente un atome d'hydrogène ou un groupe C₁₋₆-alkyle ; R₈ représente un atome d'hydrogène ou un groupe C₁₋₆-alkyle, C₃₋₇-cycloalkyle, C₃₋₇-cycloalkyle-C₁₋₃alkylène.

Parmi les composés de formule générale (I'), un second sous-groupe de composés est constitué des composés pour lesquels :
A, n et R₁ sont tels que définis dans le premier sous-groupe défini ci-dessus ;
R₇ représente un atome d'hydrogène ;
R₈ représente un atome d'hydrogène ou un groupe C₁₋₆-alkyle, plus particulièrement un méthyle.

Parmi les composés de formule générale (I'), les composés suivants peuvent être cités :
- [(5-phénylpyridin-2-yl)méthyl]carbamate de 2-(méthylamino)-2-oxoéthyle
- 2-(5-phénylpyridin-2-yl)éthylcarbamate de 2-(méthylamino)-2-oxoéthyle
- 2-(6-phénylpyridin-3-yl)éthylcarbamate de 2-(méthylamino)-2-oxoéthyle
- 2-(6-phénylpyridazin-3-yl)éthylcarbamate de 2-(méthylamino)-2-oxoéthyle
- 2-(2-phénylpyrimidin-5-yl)éthylcarbamate de 2-(méthylamino)-2-oxoéthyle
- 2-(5-phénylpyrimidin-2-yl)éthylcarbamate de 2-(méthylamino)-2-oxoéthyle
- (2-isoquinolin-7-yléthyl)carbamate de 2-(méthylamino)-2-oxoéthyle
- 2-(4-phényl-1*H*-imidazol-1-yl)éthylcarbamate de 2-(méthylamino)-2-oxoéthyle
- 2-(2-phényl-1,3-oxazol-4-yl)éthylcarbamate de 2-(méthylamino)-2-oxoéthyle
- 2-(5-phényl-1,2,4-oxadiazol-3-yl)éthylcarbamate de 2-(méthylamino)-2-oxoéthyle
- (3-pyridin-2-ylpropyl)carbamate de 2-amino-2-oxoéthyle
- (3-pyridin-3-ylpropyl)carbamate de 2-amino-2-oxoéthyle
- (3-pyridin-4-ylpropyl)carbamate de 2-amino-2-oxoéthyle
- (3-pyrimidin-5-ylpropyl)carbamate de 2-amino-2-oxoéthyle
- (3-quinolin-2-ylpropyl)carbamate de 2-amino-2-oxoéthyle
- (3-quinolin-4-ylpropyl)carbamate de 2-amino-2-oxoéthyle
- (3-isoquinolin-1-ylpropyl)carbamate de 2-amino-2-oxoéthyle
- (3-isoquinolin-4-ylpropyl)carbamate de 2-amino-2-oxoéthyle
- 3-(4-phényl-1*H*-imidazol-1-yl)propylcarbamate de 2-(méthylamino)-2-oxoéthyle
- 3-(1*H*-benzimidazol-1-yl)propylcarbamate de 2-(méthylamino)-2-oxoéthyle
- (4-pyridin-2-ylbutyl)carbamate de 2-amino-2-oxoéthyle
- (4-pyridin-3-ylbutyl)carbamate de 2-amino-2-oxoéthyle
- (4-pyridin-4-ylbutyl)carbamate de 2-amino-2-oxoéthyle
- (4-pyrimidin-5-ylbutyl)carbamate de 2-amino-2-oxoéthyle
- (4-quinolin-2-ylbutyl)carbamate de 2-amino-2-oxoéthyle
- (4-quinolin-4-ylbutyl)carbamate de 2-amino-2-oxoéthyle
- (4-isoquinolin-1-ylbutyl)carbamate de 2-amino-2-oxoéthyle
- (4-isoquinolin-4-ylbutyl)carbamate de 2-amino-2-oxoéthyle
- 4-(1*H*-benzimidazol-1-yl)butylcarbamate de 2-(méthylamino)-2-oxoéthyle
- 4-(1*H*-indol-1-yl)butylcarbamate de 2-amino-2-oxoéthyle
- 4-(1*H*-indol-1-yl)butylcarbamate de 2-(méthylamino)-2-oxoéthyle
- 4-(1*H*-pyrrolo[2,3-b]pyridin-1-yl)butylcarbamate de 2-amino-2-oxoéthyle
- 4-(1*H*-pyrrolo[2,3-b]pyridin-1-yl)butylcarbamate de 2-(méthylamino)-2-oxoéthyle
- 4-(3,4-dihydroisoquinolin-2(1*H*)-yl)butylcarbamate de 2-(méthylamino)-2-oxoéthyle
- 4-(2-oxo-3,4-dihydroquinolin-1(2*H*)-yl)butylcarbamate de 2-amino-2-oxoéthyle
- 4-(2-oxo-3,4-dihydroquinolin-1(2*H*)-yl)butylcarbamate de 2-(méthylamino)-2-oxoéthyle
- (5-pyridin-2-ylpentyl)carbamate de 2-amino-2-oxoéthyle
- (5-pyridin-4-ylpentyl)carbamate de 2-amino-2-oxoéthyle
- (5-pyrimidin-5-ylpentyl)carbamate de 2-amino-2-oxoéthyle
- (5-quinolin-2-ylpentyl)carbamate de 2-amino-2-oxoéthyle
- (5-quinolin-4-ylpentyl)carbamate de 2-amino-2-oxoéthyle
- (5-isoquinolin-1-ylpentyl)carbamate de 2-amino-2-oxoéthyle
- (5-isoquinolin-4-ylpentyl)carbamate de 2-amino-2-oxoéthyle
- (3-naphthalèn-1-ylprop-2-yn-1-yl) carbamate de 2-amino-2-oxoéthyle
- (3-naphthalèn-1-ylprop-2-yn-1-yl)carbamate de 2-(méthylamino)-2-oxoéthyle
- (5-naphthalèn-1-ylpent-4-yn-1-yl)carbamate de 2-amino-2-oxoéthyle
- (5-naphthalèn-1-ylpent-4-yn-1-yl)carbamate de 2-(méthylamino)-2-oxoéthyle.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases
ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₃ une chaîne carbonée qui peut avoir de 1 à 3 atomes de carbone;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tertbutyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcènylène, un groupe aliphatique à 2 carbones, insaturé divalent, plus particulièrement un éthylène ;
- C₂-alcynylène, un groupe -C≡C- ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ;
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon différentes méthodes, illustrées par les schémas qui suivent.

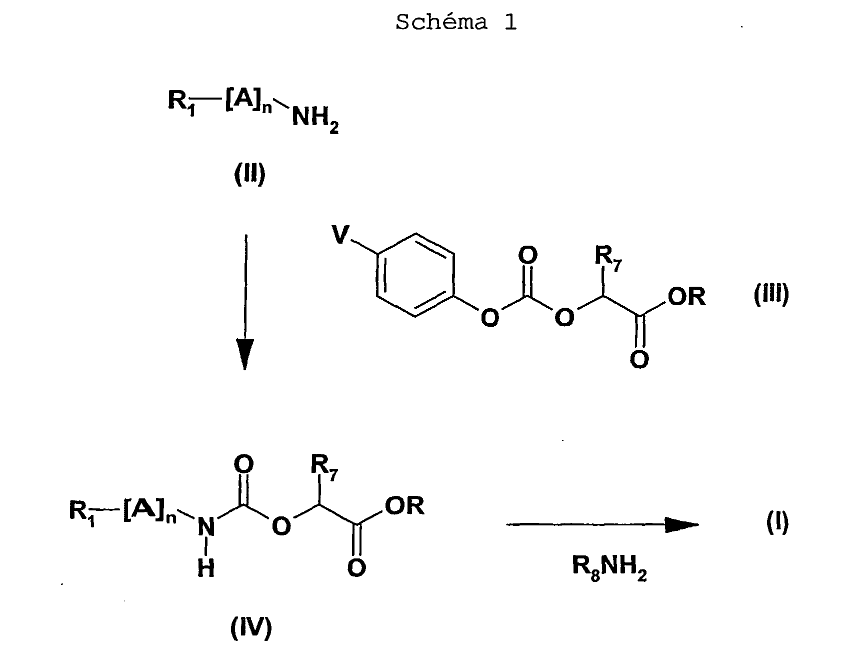

Une première méthode (schéma 1) d'obtention des composés de formule générale (I), consiste à faire réagir une amine de formule générale (II), dans laquelle R₁, A et n sont tels que définis dans la formule générale (I), avec un carbonate de formule générale (III) dans laquelle V représente un atome d'hydrogène ou un groupe nitro, R₇ est tel que défini dans la formule générale (I) et R représente un groupe méthyle ou éthyle. Le carbamate-ester de formule générale (IV) ainsi obtenu est ensuite transformé en composé de formule générale (I), par aminolyse au moyen d'une amine de formule générale R₈NH₂ où R₈ est tel que défini dans la formule générale (I). La réaction d'aminolyse peut être réalisée dans un solvant tel que le méthanol ou l'éthanol, ou un mélange de solvants tels que le méthanol et le tétrahydrofurane.

Une autre méthode (schéma 2) d'obtention des composés de formule générale (I), consiste à faire réagir un dérivé de formule générale (V) dans laquelle R₁, n et A sont tels que définis dans la formule générale (I) et W représente un groupement hydroxy, mésylate, tosylate, ou un atome de chlore, de brome ou d'iode, avec une oxazolidine-dione de structure générale (VI) dans laquelle R₇ est tel que défini dans la formule générale (I) pour fournir le dérivé oxazolidine-dione de structure générale (VII).
Dans le cas où W représente un groupement hydroxy, la réaction peut être conduite suivant les conditions de Mitsunobu (Synthesis 1981, 1-28), par exemple, par action de l'azodicarboxylate de diéthyle ou diisopropyle en présence de triphénylphosphine. Dans le cas où W représente un atome de chlore, de brome, ou d'iode, ou un groupement mésylate ou tosylate, la réaction peut être conduite en présence d'une base telle que la 1,1,3,3-tétraméthylguanidine, l'hydrure de sodium ou le tert-butoxyde de sodium dans un solvant tel que le tétrahydrofurane, l'acétonitrile ou le diméthylformamide à une température comprise entre 0°C et 80°C. Le dérivé oxazolidine-dione de formule générale (VII) ainsi obtenu est ensuite transformé en composé de formule générale (I), par aminolyse au moyen d'une amine de formule générale R₈NH₂ où R₈ est tel que défini dans la formule générale (I).

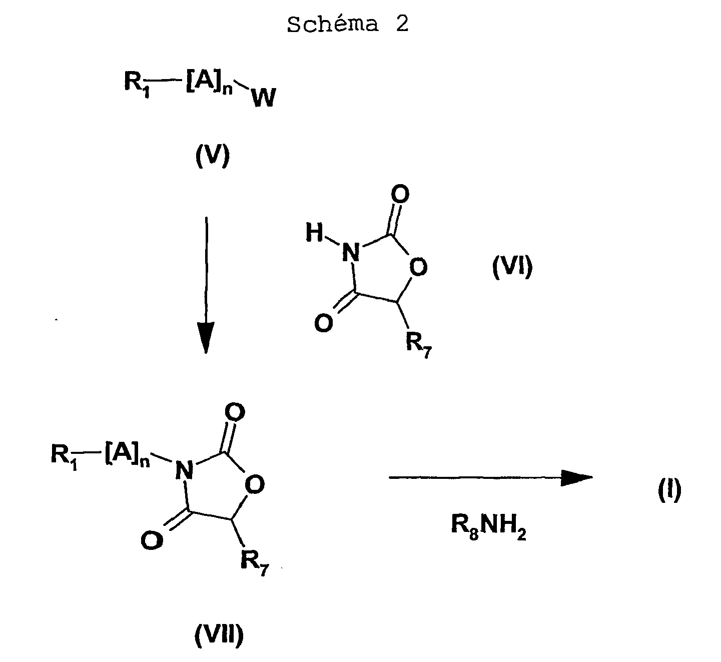

Les composés de formule générale (I), (IV) et (VII), dans laquelle R₁ représente un groupe de type aryle-aryle, aryle-hétéroaryle, hétéroaryle-aryle ou hétéroaryle-hétéroaryle, peuvent être également préparés par réaction des composés de formule générale (I), (IV) ou (VII) correspondants, pour lesquels R₂ est substitué par un atome de chlore, de brome, d'iode ou par un groupement triflate dans la position où le groupe R₄ doit être introduit, avec un dérivé d'acide aryl- ou hétéroaryl-boronique suivant les conditions de réaction de Suzuki (Chem. Rev. 1995, 95, 2457-2483) ou avec un dérivé d'aryl- ou d'hétéroaryl-tri-alkylstannane suivant les conditions de réaction de Stille (Angew. Chem. Int. Ed. 1986, 25, 504-524).

Les carbonates de formule générale (III) peuvent être préparés selon toute méthode décrite dans la littérature, par exemple par réaction d'un alcool de formule générale HOCHR₅COOR où R représente un groupe méthyle ou éthyle avec le chloroformiate de phényle ou de 4-nitrophényle, en présence d'une base telle que la triéthylamine ou la diisopropyléthylamine.

Les composés de formule générale (II), (V) et (VI) ainsi que les amines de formule générale R₈NH₂, quand leur mode de préparation n'est pas décrit, sont disponibles dans le commerce ou décrits dans la littérature, ou peuvent être préparés selon différentes méthodes décrites dans la littérature ou connues de l'homme du métier.

Les composés de formule générale (IV) dans laquelle n, A, R₁ et R₇ sont tels que définis dans la formule générale (I) et R représente un groupe méthyle ou éthyle sont nouveaux et font également partie de l'invention, les composés suivants étant exclus :
- 2-[({ [2-(5-hydroxy-1H-indol-3-yl)ethyl]amino}carbonyl)-oxy]propanoate d'éthyle ;
- 2-[({[2-[5-(phenylmethoxy)-1H-indol-3-yl]ethyl}amino)-carbonyl]oxy]propanoate d'éthyle.
   Les composés de formule générale (IV) sont utiles en tant qu'intermédiaires de synthèse pour la préparation des composés de formule générale (I).

Les composés de formule générale (VII) dans laquelle n, A, R₁ et R₇ sont tels que définis dans la formule générale (I), sont nouveaux et font également partie de l'invention, les composés suivants étant exclus :
- iodure de 2-[2-(2,4 -dioxo-3-oxazolidinyl)éthyl]-1-méthylpyridinium ;
- iodure de 2-[2-(2,4-dioxo-3-oxazolidinyl)éthyl]-5-éthyl-1-méthylpyridinium ;
- iodure de 4-[2-(2,4-dioxo-3-oxazolidinyl)éthyl]-1-méthylpyridinium ;
- chlorhydrate de 5-méthyl-3-[2-(4-pyridinyl)éthyl]-2,4-oxazolidinedione;
- chlorhydrate de 5-méthyl-3-[2-(2-pyridinyl)éthyl]-2,4-oxazolidinedione;
- 3-(5-imidazo[12-a]pyridin-5-ylpentyl)-2,4-oxazolidinedione;
- 3-[2-(5-méthyl-4-thiazolyl)éthyl]-2,4-oxazolidinedione;
- 3-[2-(1H-pyrrol-2-yl)éthyl]-2,4-oxazolidinedione;
- 3-[2-(2-thiènyl)éthyl]-2,4-oxazolidinedione;
- 3-[3-(2-thiènyl)propyl]-2,4-oxazolidinedione;
- 3-[4-(2-thiènyl)butyl]-2,4-oxazolidinedione;
- 5-méthyl-3-[2-(2-thiènyl)éthyl]-2,4-oxazolidinedione;
- 5-éthyl-3-[2-(2-thiènyl)éthyl]-2,4-oxazolidinedione;
- 3-[2-(3-thiènyl)éthyl]-2,4-oxazolidinedione;
- 3-[2-(5-méthyl-2-thiènyl)éthyl]-2,4-oxazolidinedione;
- 3-[2-(5-acétyl-2-thiènyl)éthyl]-2,4-oxazolidinedione;
- 3-[2-(5-bromo-2-thiènyl)éthyl]-2,4-oxazolidinedione;
- 5-[2-(2,4-dioxo-3-oxazolidinyl)éthyl]-2-thiophènecarboxaldéhyde;
- 3-[3-(1-indolinyl)propyl]-2,4-oxazolidinedione;
- 3-[3-(1-indolinyl)propyl]-5-méthyl-2,4-oxazolidinedione;
- 3-[2-(2-pyridinyl)éthyl]-2,4-oxazolidinedione;
- 3-[2-(5-éthyl-2-pyridinyl)éthyl]-5-méthyl-2,4-oxazolidinedione;
- 5-éthyl-3-[2-(5-éthyl-2-pyridinyl)éthyl]-2,4-oxazolidinedione;
- 3-[2-(5-éthyl-2-pyridinyl)éthyl]-5-isopropyl-2,4-oxazolidinedione;
- 3-[2-(4-pyridinyl)éthyl]-2,4-oxazolidinedione;
- 5-éthyl-3-[2-(4-pyridinyl)éthyl]-2,4-oxazolidinedione;
- 5-éthyl-3-[2-(2-pyridinyl)éthyl]-2,4-oxazolidinedione;
- 5-isopropyl-3-[2-(4-pyridinyl)éthyl]-2,4-oxazolidinedione;
- 5-isopropyl-3-[2-(2-pyridinyl)éthyl]-2,4-oxazolidinedione;
- 3-[2-(5-éthyl-2-pyridinyl)éthyl]-2,4-oxazolidinedione.
Les composés de formule générale (VII) sont utiles en tant qu'intermédiaires de synthèse pour la préparation des composés de formule générale (I).

Les composés exclus des formules générales (IV) et (VII) telles que définies ci-dessus, sont divulgués dans les documents suivants : US3,876,661 ; US3,054,794 ; US2,866,734 ; WO95/35296; JP60163881; JP60174766; JP59216885; Kobayashi, Michihiro et al, Yakugaku Zasshi, 1984, 104(6), 680-90.

Un sous-groupe de composés de formule générale (VII) est constitué des composés pour lesquels :
n, A, R₁ et R₇ sont tels que définis dans la formule générale (I) à la condition que :
   - quand R₂ est un groupe pyrrolyle, imidazo[1,2-a]pyridinyle ou indolinyle alors R₂ est substitué par un ou plusieurs groupes R₃ et/ou R₄ ;
   - quand R₂ est un groupe pyridinyle, alors R₂ est substitué par un ou plusieurs groupes R₃ et/ou R₄ où R₃ est différent d'un méthyle ou d'un éthyle ;
   - quand R₂ est un groupe thiènyle, alors R₂ est substitué par un ou plusieurs groupes R₃ et/ou R₄ où R₃ est différent d'un brome, d'un méthyle ou d'un groupe CHO ou COCH₃ ;
   - quand R₂ est un groupe thiazolyle substitué par un groupe R₃, alors R₃ est différent d'un méthyle.

Les exemples qui vont suivre illustrent la préparation de quelques composés de l'invention. Ces exemples ne font qu' illustrer l'invention. Les microanalyses, les spectres I.R. et R.M.N. et/ou la LC-MS (Liquid Chromatography coupled to Mass Spectroscopy) confirment les structures et les puretés des composés obtenus.
PF(°C) représente le point de fusion en degrés Celsius.
Les numéros indiqués entre parenthèses dans les titres des exemples correspondent à ceux de la 1ère colonne du tableau ci-après.

### Exemple 1 (composé n°3)

### 2-(6-phénylpyridin-3-yl)éthylcarbamate de 2-méthylamino)-2-oxoéthyle

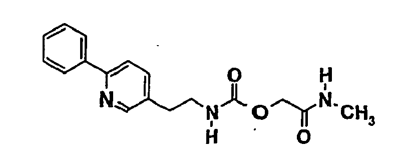

1.1. 2-(6-phénylpyridin-3-yl)éthylcarbamate de phénylméthyle A une solution de 4,5 g (25,60 mmoles) d'éthènylcarbamate de phénylméthyle (Org. Proc. Res. & Develop. ; 2002, 6, 74-77) dans 25 ml de tétrahydrofurane, refroidie à environ-10°C, on ajoute goutte à goutte, sous atmosphère inerte, une solution de 3,12 g (12,80 mmoles) de 9-borabicyclo[3.3.1]nonyle (BBN) dans 100 ml de tétrahydrofurane tout en maintenant la température du milieu réactionnel inférieur à -10°C. On poursuit l'agitation à -10°C pendant 1 heure, puis à température ambiante pendant 4 heures.
On ajoute 18 ml d'une solution aqueuse d'hydroxyde de sodium (3N) et on poursuit l'agitation pendant 1 heure. On ajoute ensuite 4,0 g (17,1 mmoles) de 5-bromo-2-phénylpyridine et 2, 12 g (2,6 mmoles) de PdCl₂ (dppf).CH₂Cl₂ (dppf : 1,1'-bis(diphénylphosphino)-ferrocène). On poursuit l'agitation à température ambiante pendant 18 heures.
On refroidit le mélange réactionnel par un bain d'eau glacée puis on additionne goutte à goutte 40 ml d'un mélange 2/1 d'une solution tampon (pH=7) et d'eau oxygénée à 30 %. On laisse sous agitation à température ambiante pendant 1 heure. On sépare la phase aqueuse puis on l'extrait trois fois avec du dichlorométhane. On réunit les phases organiques et on les lave successivement avec de l'eau et une solution aqueuse saturée en chlorure de sodium. On sèche la phase organique sur sulfate de sodium et on concentre le filtrat sous pression réduite. On purifie le résidu ainsi obtenu par chromatographie sur gel de silice en éluant avec un mélange 20/80 d'acétate d'éthyle et de cyclohexane.
On obtient 2,9 g de produit sous forme de solide blanc. PF (°C) : 118°C

### 1.2. 2-(6-phénylpyridin-3-yl)éthanamine

A une solution de 1,8 g (5,42 mmoles) de 2-(6-phénylpyridin-3-yl)éthylcarbamate de phénylméthyle, préparé à l'étape 1.1., dans 50 ml de dichlorométhane, refroidie à environ 0°C, on ajoute goutte à goutte 9 ml d'acide bromhydrique à 33% dans l'acide acétique. On poursuit l'agitation à température ambiante pendant 2 heures. On concentre sous pression réduite, on reprend le résidu avec du dichlorométhane et une solution aqueuse saturée d'hydrogénocarbonate de sodium. On sépare la phase aqueuse et on l'extrait deux fois avec de l'acétate d'éthyle. On lave les phases organiques réunies avec une solution aqueuse saturée en chlorure de sodium, on les sèche sur sulfate de sodium et on concentre le filtrat sous pression réduite.
On obtient 0,86 g de produit sous forme d'huile utilisé tel quel dans l'étape suivante.

### 1.3. ({[2-(6-phénylpyridin-3-yl)éthyl]amino}carbonyl)-oxyacétate d'éthyle

On chauffe à 60°C pendant 12 heures, une solution de 0,85 g (4,29 mmoles) de 2-(6-phénylpyridin-3-yl)éthanamine, préparé à l'étape 1.2., et de 1,25 g (5,58 mmoles) de [(phényloxycarbonyl)oxy]acétate d'éthyle (J- Med. Chem., 1999, 42, 277-290) dans 40 ml de toluène. On laisse revenir à température ambiante, on sépare l'insoluble par filtration et on concentre le filtrat sous pression réduite. On purifie le résidu ainsi obtenu par chromatographie sur gel de silice en éluant avec un mélange 30/70 d'acétate d'éthyle et de cyclohexane.
On obtient ainsi 1,06 g de produit sous forme d'huile.

### 1.4. 2-(6-phénylpyridin-3-yl)éthylcarbamate de 2-(méthylamino)-2-oxoéthyle

A une solution de 1,0 g (3,06 mmoles) de ({[2-(6-phénylpyridin-3-yl)éthyl]amino}carbonyl)oxyacétate d'éthyle, obtenu à l'étape 1.3., dans 6 ml de méthanol, on ajoute 4,6 ml (9,17 mmoles) d'une solution de méthylamine (2M) dans le tétrahydrofurane. On poursuit l'agitation à température ambiante pendant 4 heures.
Après concentration sous pression réduite, on purifie le résidu obtenu par chromatographie sur gel de silice en éluant avec un mélange 95/5 de dichlorométhane et de méthanol. On obtient un solide blanc que l'on recristallise dans l'acétate d'éthyle.
On obtient 0,510 g de produit sous forme de solide blanc. LC-MS : M+H = 314
PF(°C) : 130-132°C
RMN¹H (CDCl₃) δ (ppm) : 2,85 (d, 3H) ; 2,95 (t, 2H) ; 3,55 (q, 2H) ; 4,60 (s, 2H) ; 5,05 (large s, 1H) ; 6,10 (large s, 1H) ; 7,50 (m, 3H) ; 7,70 (m, 2H) ; 8,0 (d, 2H) ; 8,60 (s, 1H).

### Exemple 2 (composé n° 56)

### 4-(1H-indol-1-yl)butylcarbamate de 2-(méthylamino)-2-oxoéthyle

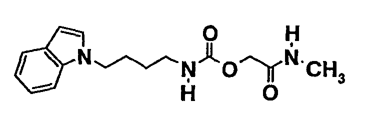

### 2.1. 1-(4-bromobutyl)-1H-indole

A une solution de 2 g (17,07 mmoles) de 1H-indole et de 1,15 ml (51,22 mmoles) de 1,4-dibromobutane dans 80 ml de diméthylformamide, refroidie par un bain d'eau glacée, on ajoute par petites portions 0,96 g (17,07 mmoles) d'hydroxyde de sodium. On enlève le bain et on poursuit l'agitation à température ambiante pendant 15 heures.
Après concentration sous pression réduite, on reprend le résidu par de l'eau et de l'acétate d'éthyle. On sépare la phase aqueuse, on l'extrait deux fois avec de l'acétate d'éthyle, on lave les phases organiques réunies avec une solution aqueuse saturée en chlorure de sodium, on les sèche sur sulfate de sodium et on concentre le filtrat sous pression réduite. On purifie le résidu ainsi obtenu par chromatographie sur gel de silice en éluant avec un mélange 1/99 d'acétate d'éthyle et de cyclohexane.
On obtient 3 g de produit sous forme d'huile jaune.

### 2.2. 3-[4-(1H-indol-1-yl)butyl]-1,3-oxazolidine-2,4-dione

On porte au reflux pendant 14 heures, sous atmosphère inerte, une solution de 3 g (11,90 mmoles) de 1-(4-bromobutyl)-1H-indole, préparé à l'étape 2.1., de 2,41 g (23,80 mmoles) de 1,3-oxazolidin-2,4-dione (J. Med. Chem., 1991, 34, 1538-1544) et de 2,74 g (23,80 mmoles) de 1,1,3,3-tétraméthylguanidine, dans 30 ml de tétrahydrofurane.
On concentre sous pression réduite. On reprend le résidu par de l'acétate d'éthyle et de l'eau, on sépare la phase aqueuse, on l'extrait deux fois avec de l'acétate d'éthyle, on lave les phases organiques réunies avec une solution aqueuse saturée en chlorure de sodium et on les sèche sur sulfate de sodium.
Après évaporation du solvant, le résidu obtenu est purifié par chromatographie sur gel de silice en éluant avec un mélange 10/90 puis 20/80 d'acétate d'éthyle et de cyclohexane.
On obtient 2 g de produit sous forme de solide blanc.

### 2.3. 4-(1H-indol-1-yl) butylcarbamate de 2-(méthylamino)-2-oxoéthyle

On procède comme décrit dans l'exemple 1 (étape 1.4.). A partir de 0,90 g (3,31 mmoles) de 3-[4-(1H-indol-1-yl)butyl]-1,3-oxazolidine-2,4-dione, obtenu à l'étape 2.2., et de 5 ml (9,93 mmoles) d'une solution de méthylamine (2M) dans le tétrahydrofurane, et après chromatographie sur gel de silice en éluant avec un mélange 98/2 de dichlorométhane et de méthanol, on obtient 0,70 g de produit sous forme de solide blanc amorphe.
LC-MS : M+H = 304
PF(°C) : 64-67°C
RMN¹H (CDCl₃) δ (ppm) : 1,50 (m, 2H) ; 1,90 (m, 2H) ; 2, 80 (d, 3H) ; 3,20 (q, 2H) ; 4,20 (t, 2H) ; 4,55 (s, 2H) ; 5,95 (large s, 1H) ; 6,10 (large s, 1H) ; 6,50 (d, 1H) ; 7,20 (m, 3H) ; 7,40 (d, 1H) ; 7,70 (d, 1H) -

### Exemple 3 (composé n° 71)

### (5-naphtalèn-1-ylpent-4-yn-1-yl) carbamate de 2-amino-2-oxoéthyle

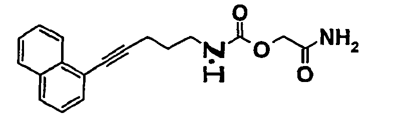

### 3.1. 5-naphtalèn-1-ylpent-4-yn-1-ol

Sous atmosphère d'argon, on ajoute goutte à goutte une solution de 0,59 g (7 mmoles) de 4-pentyn-1-ol dans 3 ml d'acétonitrile à une suspension de 1,78 g (7 mmoles) de 1-iodonaphtalène, de 1,48 ml (10,5 mmoles) de triéthylamine, de 0,066 g (0,35 mmole) d'iodure cuivreux et de 0,147 g (0,21 mmole) de dichlorure de di(triphénylphosphine)palladium (Ph₃P)₂PdCl₂ dans 4 ml d'acétonitrile. On agite 3 heures à température ambiante puis on ajoute 4 g de silice et on évapore à sec. On purifie le produit par chromatographie sur gel de silice en éluant par un mélange 80/20 puis 60/40 de cyclohexane et d'acétate d'éthyle pour obtenir 1,22 g de produit sous forme d'huile jaune.

### 3.2. méthanesulfonate de 5-naphtalèn-1-ylpent-4-yn-1-yle

A une solution de 1,2 g (5,71 mmoLes) de 5-naphtalèn-1-ylpent-4-yn-1-ol obtenu à l'étape 3.1, et de 1,6 ml (11,4 mmoles) de triéthylamine dans 12 ml de dichlorométhane, refroidie par un bain de glace, on ajoute goutte à goutte une solution de 0,85 g (7,42 mmoles) de chlorure de méthanesulfonyle dans 5 ml de dichlorométhane. On agite 1 heure à 0°C puis 2 heures à température ambiante. On ajoute ensuite 25 ml d'eau et 75 ml de dichlorométhane. On décante la phase organique, on la lave par 25 ml d'eau puis par 25 ml d'une solution aqueuse saturée en chlorure de sodium. On la sèche sur sulfate de magnésium et on l'évapore à sec pour obtenir 1, 68 g de produit sous forme d'huile orange, utilisé directement dans l'étape suivante.

### 3.3. 3-(5-naphtalèn-1-ylpent-4-yn-1-yl)-1,3-oxazolidine-2,4-dione.

On dissout 1,64 g (5,70 mmoles) de méthanesulfonate de 5-naphtalèn-1-ylpent-4-yn-1-yle obtenu à l'étape 3.2, et 0,72 g (7,1 mmoles) de 1,3-oxazolidine-2,4-dione dans 9 ml de tétrahydrofurane. On ajoute 1,3 g (11,4 mmoles) de 1,1,3,3-tétraméthylguanidine en solution dans 3 ml de tétrahydrofurane. On chauffe à reflux pendant une nuit. On ajoute 25 ml d'acétate d'éthyle et 6 g de silice. On évapore à sec. On purifie le résidu par chromatographie sur gel de silice en éluant par un mélange 90/10 puis 80/20 et 70/30 de cyclohexane et d'acétate d'éthyle pour obtenir 1,33 g de produit sous forme de solide blanc.
PF(°C) : 99-101°C

### 3.4. (5-naphtalèn-1-ylpent-4-yn-1-yl)carbamate de 2-amino-2-oxoéthyle

On dissout 0,75 g (2,56 mmoles) de 3-(5-naphtalèn-1-ylpent-4-yn-1-yl)-1,3-oxazolidine-2,4-dione obtenu à l'étape 3.3, dans 18 ml d'une solution 7M d'ammoniac (126 mmoles) dans le méthanol. On laisse une nuit à température ambiante. On ajoute ensuite 3 g de silice et on évapore à sec. On purifie le résidu par chromatographie sur gel de silice en éluant par un mélange 98/2 puis 96/4 et 94/6 de dichlorométhane et de méthanol. On recristallise ensuite dans un mélange d'acétate d'éthyle et de diisopropyléther pour obtenir 0,59 g de produit sous forme de cristaux blancs.
LC-MS : M+H = 311
PF(°C) : 105-108°C
RMN¹H (CDCl₃) δ (ppm) : 8,33 (d, 1H) ; 7,85 (m, 2H) ; 7,70-7,40 (m, 4H); 5,90 (m large, 1H); 5,50 (m large 1H); 5,20 (m large 1H) ; 4,55 (s, 2H); 3,50 (m, 2H); 2,70 (t, 2H); 1,95 (m, 2H).

### Exemple 4 (composé n°29)

### (3-[3-(4-chlorophényl)isoxazol-5-yl]propyl)carbamate de 2-(méthylamino)-2-oxoéthyle

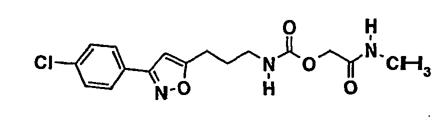

### 4.1. 3-[3-(4-chlorophényl)isoxazol-5-yl]propan-1-ol

On ajoute goutte à goutte 1,6 ml (11,5 mmoles) de triéthylamine à une solution de 1,18 ml (12,6 mmoles) de pent-4-yn-1-ol et de 2,0 g (10,5 mmoles) de chlorure de 4-chloro-N-hydroxybenzenecarboximidoyle(J.Med.Chem.19 98,41,4556-4566) dans 30 ml de dichlorométhane refroidie par un bain de glace. On laisse réagir une nuit à température ambiante. On ajoute 50 ml de dichlorométhane et 70 ml d'eau. On sépare la phase organique et on extrait la phase aqueuse par 2 fois 50 ml de dichlorométhane. Les phases organiques sont ensuite lavées par 2 fois 70 ml d'eau puis par 70 ml d'une solution aqueuse saturée en chlorure de sodium, séchées sur sulfate de sodium et évaporées. On purifie le résidu par chromatographie sur gel de silice en éluant par un mélange de cyclohexane et d'acétate d'éthyle pour obtenir 1,3 g (5,47 mmoles) de produit sous forme de solide blanc.
PF(°C) : 60-62°C

### 4.2. 3-{3-[3-(4-chlorophényl)isoxazol-5-yl]propyl}-1,3-oxazolidine-2,4-dione

A une solution de 1,30 g (5,47 moles) de 3-[3-(4-chlorophényl) isoxazol-5-yl]propan-1-ol, préparé à l'étape 4.1., et de 0,9 ml (7,11 mmoles) de triéthylamine dans 70 ml de dichlorométhane, refroidie par un bain de glace, on ajoute goutte à goutte 0,5 ml (6,0 mmoles) de chlorure de méthanesulfonyle. On agite ensuite 2 heures à température ambiante. On ajoute 70 ml d'eau et on sépare la phase organique. On extrait la phase aqueuse par 2 fois 70 ml de dichlorométhane . Les phases organiques sont ensuite lavées par 100 ml d'eau et 100 mL d'une solution aqueuse saturée en chlorure de sodium, séchées sur sulfate de sodium et évaporées.
On redissout le résidu dans 60 ml de tétrahydrofurane et on ajoute 0,9 g (8,9 mmoles) de 1,3-oxazolidine-2,4-dione et 1,1 ml (8,7 mmoles) de 1,1,3,3-tétraméthylguanidine. On chauffe à 65°C pendant une nuit. On reprend dans un mélange de 100 ml d'eau et de 100 ml d'acétate d'éthyle. On sépare la phase organique et on extrait la phase aqueuse par 2 fois 80 ml d'acétate d'éthyle. On lave les phases organiques par 100 ml d'eau puis 100 ml d'une solution aqueuse saturée en chlorure de sodium, on sèche sur sulfate de sodium et on évapore. On purifie le résidu par chromatographie sur gel de silice en éluant par un mélange 90,5/0,5 de dichlorométhane et de méthanol pour obtenir 1,0 g (3,1 mmoles) de produit sous forme de solide blanc.

### 4.3. {3-[3-(4-chlorophényl)isoxazol-5-yl]propyl}carbamate de 2-(méthylamino)-2-oxoéthyle

On dissout 0,6 g (1,87 mmoles) de 3-{3-[3-(4-chlorophényl)isoxazol-5-yl]propyl}-1,3-oxazolidine-2,4-dione, préparé à l'étape 4.2., dans un mélange de 8 ml de tétrahydrofurane et de 15 ml de méthanol. On ajoute 2,8 ml d'une solution 2M de méthylamine (5,6 mmoles) dans le tétrahydrofurane. On laisse réagir une nuit à température ambiante puis on évapore. On purifie le résidu par chromatographie sur gel de silice en éluant par un mélange 98/2 puis 95/5 et 90/10 de dichlorométhane et de méthanol. On recristallise dans un mélange d'acétate d'éthyle et de méthanol pour obtenir 0,49 g (1,4 mmoles) de cristaux blancs.
LC-MS : M+H = 352
PF(°C) : 158-160°C
RMN¹H (CDCl₃) δ (ppm) : 7,75 (d, 2H) ; 7,45 (d, 2H) ; 6,35 (s, 1H) ; 6,10 (large s, 1H) ; 5,00 (large s, 1H) ; 4,60 (s, 2H) ; 3,35 (m, 2H) ; 2,85 (m+d, 5H) ; 2,05 (m, 2H).

### Exemple 5 (composé n°20)

### (3-[6-(4-chlorophényl)pyrimidin-4-yl]propyl)carbamate de 2-(méthylamino)-2-oxoéthyle

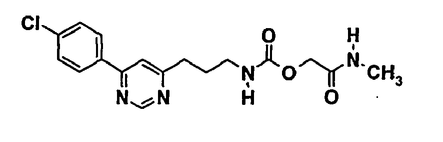

### 5.1. 1-(4-chlorophényl)-6-(tétrahydro-2H-pyran-2-yloxy)hex-2-yn-1-ol

A une solution de 13,25 g (78,8 mmoles) de 2-(pent-4-yn-1-yloxy)tétrahydro-2*H*-pyrane dans 130 mL de tétrahydrofurane anhydre refroidie à -78°C sous argon, on ajoute goutte à goutte 61,5 ml d'une solution de n-butyllithium 1,6 M (98,4 mmoles) dans l'hexane. On poursuit l'agitation pendant 1 heure à -78°C puis on ajoute goutte à goutte une solution de 12,18 g (86,6 mmoles) de 4-chlorobenzaldéhyde dans 40 ml de tétrahydrofurane. On poursuit l'agitation pendant 2 heures à -78°C puis on réchauffe la solution à 0°C et on la verse sur sur 300 ml d'une solution aqueuse saturée en chlorure d'ammonium. On extrait par 450 ml d'acétate d'éthyle. On lave la phase organique par 50 ml d'eau puis 50 ml d'une solution aqueuse saturée en chlorure de sodium. On sèche sur sulfate de sodium et on évapore. On purifie le résidu par chromatographie sur gel de silice en éluant par un mélange 70/30 de n-hexane et d'acétate d'éthyle pour obtenir 16,64 g (53,88 mmoles) de produit sous forme d'huile incolore.

### 5.2. 1-(4-chlorophényl)-6-(tétrahydro-2H-pyran-2-yloxy) hex-2-yn-1-one

On ajoute goutte à goutte une solution de 16,60 g (53,7 mmoles) de 1-(4-chlorophényl)-6-(tétrahydro-2*H*- pyran-2-yloxy)hex-2-yn-1-ol, préparé à l'étape 5.1., à une suspension de 93 g (1,07 moles) de dioxyde de manganèse dans 500 ml de dichlorométhane refroidie par un bain de glace. On poursuit l'agitation pendant 1,5 heure puis on filtre sur célite et on rince par du dichlorométhane. On évapore les filtrats pour obtenir 16,3 g (53,1 mmoles) de produit sous forme d'huile jaunâtre.

### 5.3. 4-(4-chlorophényl)-6-[3-(tétrahydro-2H-pyran-2-yloxy)propyl]pyrimidine

On agite à 40°C pendant 5 heures un mélange de 3,60 g (11, 73 mmoles) de 1- (4-chlorophényl) -6- (tétrahydro-2*H*-pyran-2-yloxy)hex-2-yn-1-one, préparé à l'étape 5.2., de 9,45 g (117 mmoles) de chlorhydrate de formamidine et de 25 g (234 mmoles) de carbonate de sodium en suspension dans 108 ml d'acétonitrile et 0,1 ml d'eau. On reprend ensuite par 600 ml d'eau et 400 ml d'une solution aqueuse saturée en carbonate de sodium. On décante la phase organique, on la lave par 200 mL d'eau et 200 ml d'une solution aqueuse saturée en chlorure de sodium, on la sèche sur sulfate de sodium et on l'évapore. On purifie le résidu par chromatographie sur gel de silice en éluant par un mélange 70/30 de n-hexane et d'acétate d'éthyle pour obtenir 3,22 g (9,67 mmoles) de produit sous forme d'huile jaunâtre.

### 5.4. 3-[6-(4-chlorophényl)pyrimidin-4-yl]propan-1-ol

On dissout 3,22 g (9,67 mmoles) de 4-(4-chlorophényl)-6-[3 - (tétrahydro-2*H*-pyran-2-yloxy)propyl] pyrimidine, préparé à l'étape 5.3., dans 32 ml de méthanol et on ajoute 16 ml d'une solution d'acide chlorhydrique 4N dans le dioxane. On agite 1,5 heures à température ambiante puis on ajoute par portion 150 ml d'une solution aqueuse à demi-saturée en hydrogénocarbonate de sodium. On extrait par 350 ml d'acétate d'éthyle. On lave la phase organique par 50 ml d'eau et 50 ml d'une solution aqueuse saturée en chlorure de sodium, on séche sur sulfate de sodium et on évapore pour obtenir 2,33 g (9,36 mmoles) de produit sous forme de solide blanc.
PF(°C) : 75-76°C

### 5.5. 3-{3-[6-(4-chlorophényl)pyrimidin-4-yl]propyl}-1,3-oxazolidine-2,4-dione

A une solution de 0,111 g (0,44 mmoles) de 3-[6-(4-chlorophényl)pyrimidin-4-yl]propan-1-ol, préparé à l'étape 5.4., de 0,077 g (0,76 mmoles) de 1,3-oxazolidine-2,4-dione et de 0,235 g (0,89 mmoles) de triphénylphosphine dans 4 ml de tétrahydrofurane, refroidie par un bain de glace, on ajoute 0,4 ml d'une solution à 40% de azodicarboxylate de diéthyle (0,9 mmoles) dans le toluène. On agite ensuite pendant une nuit à température ambiante. On reprend par un mélange d'acétate d'éthyle et d'eau. On lave la phase organique par une solution aqueuse saturée en chlorure de sodium, on sèche sur sulfate de sodium et on évapore. On purifie le résidu par chromatographie sur gel de silice en éluant par un mélange 97/3 de dichlorométhane et de méthanol pour obtenir 0,117 g (0,35 mmoles) de produit sous forme solide.

### 5.6. {3-[6-(4-chlorophényl)pyrimidin-4-yl]propyl}carbamate de 2-(méthylamino)-2-oxoéthyle

On redissout 0,113 g (0,34 mmoles) de 3-{3-[6-(4-chlorophényl)pyrimidin-4-yl]propyl}-1,3-oxazolidine-2,4-dione, préparé à l'étape 5.5., dans un mélange de 4 ml d'éthanol, 1 ml de méthanol et 1 ml de dichlorométhane. On ajoute 2 ml d'une solution 8M de méthylamine (16 mmoles) dans l'éthanol. On agite pendant 2 heures à température ambiante puis on évapore. On reprend le résidu solide par du diéthyléther pour obtenir 0,127 g (0,34 mmoles) de produit sous forme de solide blanc.
LC-MS : M+H = 363
PF(°C) : 147-149°C
RMN¹H (CDCl₃) δ (ppm) : 9,15 (s, 1H) ; 8,05 (d, 2H) ; 7,65 (s, 1H) ; 7,55 (d, 2H) ; 6,15 (large s, 1H) ; 5,30 (large s, 1H) ; 4,55 (s, 2H) ; 3,35 (m, 2H) ; 2,85 (m+d, 5H) ; 2,10 (m, 2H).

Le tableau 1 qui suit illustre les structures chimiques et les propriétés physiques de quelques composés selon l'invention. Dans ce tableau :
- tous les composés sont sous forme de base libre,
- i-propyl, n-butyle et t-butyle représentent respectivement des groupes isopropyle, butyle linéaire et tertiobutyle.

**Tableau 1**

| | | | | | |
|---|---|---|---|---|---|
| 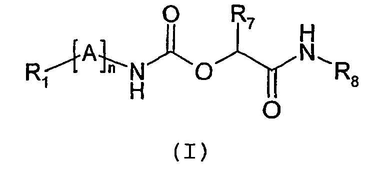 | | | | | |

| n° | R₁ | **[A]ₙ** | **R₇** | **R₈** | **PF°C (M+H)** |
|---|---|---|---|---|---|
| 1. | 5-phénylpyridin-2-yl | CH₂ | H | CH₃ | 136-138 |
| 2. | 5-phénylpyridin-2-yl | (CH₂)₂ | H | CH₃ | (314) |
| 3. | 6-phénylpyridin-3-yl | (CH₂)₂ | H | CH₃ | 130-132 |
| 4. | 6-phénylpyridazin-3-yl | (CH₂)₂ | H | CH₃ | 159-161 |
| 5. | 2-phénylpyrimidin-5-yl | (CH₂)₂ | H | CH₃ | 125-127 |
| 6. | 5-phénylpyrimidin-2-yl | (CH₂)₂ | H | CH₃ | 150-152 |
| 7. | 6-(4-Cl-phényl)-pyrimidin-4-yl | (CH₂)₂ | H | CH₃ | 139-141 |
| 8. | 6-(4-Cl-phényl)-2-méthylpyrimidin-4-yl | (CH₂)₂ | H | CH₃ | 140-142 |
| 9. | 6-(4-Cl-phényl)-2-(diméthylamino)-pyrimidin-4-yl | (CH₂)₂ | H | CH₃ | 131-133 |
| 10. | isoquinolin-7-yl | (CH₂)₂ | H | CH₃ | 134-136 |
| 11. | 4-phénylimidazol-1-yl | (CH₂)₂ | H | CH₃ | 111-113 |
| 12. | 2-phényloxazol-4-yl | (CH₂)₂ | H | CH₃ | 94-98 |
| 13. | 5-(4-Cl-phényl)-isoxazol-3-yl | (CH₂)₂ | H | CH₃ | 150-152 |
| 14. | 3-(4-Cl-phényl)-1-méthyl-1*H*-pyrazol-5-yl | (CH₂)₂ | H | CH₃ | 125-127 |
| 15. | 5-phényl-1,2,4-oxadiazol-3-yl | (CH₂)₂ | H | CH₃ | 131-135 |
| 16. | pyridin-2-yl | (CH₂)₃ | H | H | 114-115 |
| 17. | pyridin-3-yl | (CH₂)₃ | H | H | 105-107 |
| 18. | pyridin-4-yl | (CH₂)₃ | H | H | 161-162 |
| 19. | pyrimidin-5-yl | (CH₂)₃ | H | H | 119-121 |
| 20. | 6-(4-Cl-phényl)-pyrimidin-4-yl | (CH₂)₃ | H | CH₃ | 147-149 |
| 21. | 6-(4-Cl-phényl)-2-méthylpyrimidin-4-yl | (CH₂)₃ | H | CH₃ | (377) |
| 22. | 6-(4-Cl-phényl)-2-(diméthylamino)-pyrimidin-4-yl | (CH₂)₃ | H | CH₃ | 150-152 |
| 23. | quinolin-2-yl | (CH₂)₃ | H | H | 117-119 |
| 24. | quinolin-4-yl | (CH₂)₃ | H | H | 150-152 |
| 25. | isoquinolin-1-yl | (CH₂)₃ | H | H | 123-124 |
| 26. | isoquinolin-4-yl | (CH₂)₃ | H | H | 154-156 |
| 27. | 5-(4-Cl-phényl)-isoxazol-3-yl | (CH₂)₃ | H | CH₃ | 138-140 |
| 28. | 3-(4-Cl-phényl)-isoxazol-5-yl | (CH₂)₃ | H | H | 176-178 |
| 29. | 3-(4-Cl-phényl)-isoxazol-5-yl | (CH₂)₃ | H | CH₃ | 158-160 |
| 30. | 3-(4-phényl-phényl)-isoxazol-5-yl | (CH₂)₃ | H | CH₃ | 198-200 |
| 31. | 3-(naphtalèn-2-yl)-isoxazol-5-yl | (CH₂)₃ | H | CH₃ | 143-145 |
| 32. | 3-(4-Cl-phényl)1-méthyl-1*H*-pyrazol-5-yl | (CH₂)₃ | H | CH₃ | (365) |
| 33. | 4-phényl-imidazol-1-yl | (CH₂)₃ | H | CH₃ | 96-98 |
| 34. | benzimidazol-1-yl | (CH₂)₃ | H | CH₃ | 154-156 |
| 35. | pyridin-2-yl | (CH₂)₄ | H | H | 141-143 |
| 36. | pyridin-3-yl | (CH₂)₄ | H | H | 131-133 |
| 37. | pyridin-4-yl | (CH₂)₄ | H | H | 124-126 |
| 38. | pyrimidin-5-yl | (CH₂)₄ | H | H | 139-141 |
| 39. | 6-(4-Cl-phényl)-pyrimidin-4-yl | (CH₂)₄ | H | CH₃ | 101-103 |
| 40. | 6-(4-Cl-phényl)-2-méthylpyrimidin-4-yl | (CH₂)₄ | H | CH₃ | 118-120 |
| 41. | 6-(4-Cl-phényl)-2-cyclopropylpyrimidin-4-yl | (CH₂)₄ | H | CH₃ | 118-120 |
| 42. | 6-(4-Cl-phényl)-2-(diméthylamino)-pyrimidin-4-yl | (CH₂)₄ | H | CH₃ | 120-122 |
| 43. | quinolin-2-yl | (CH₂)₄ | H | H | 131-133 |
| 44. | quinolin-4-yl | (CH₂)₄ | H | H | (302) |
| 45. | isoquinolin-1-yl | (CH₂)₄ | H | H | 119-121 |
| 46. | isoquinolin-4-yl | (CH₂)₄ | H | H | 154-156 |
| 47. | 5-(4-Cl-phényl)-isoxazol-3-yl | (CH₂)₄ | H | CH₃ | 130-132 |
| 48. | 3-(4-Cl-phényl)-isoxazol-5-yl | (CH₂)₄ | H | CH₃ | 138-140 |
| 49. | 3-(4-phényl-phényl)-isoxazol-5-yl | (CH₂)₄ | H | CH₃ | 193-195 |
| 50. | 3-(naphtalèn-2-yl)-isoxazol-5-yl | (CH₂)₄ | H | CH₃ | 158-160 |
| 51. | 3-(4-Cl-phényl)1-méthyl-1*H* pyrazol-5-yl | (CH₂)₄ | H | CH₃ | 144-146 |
| 52. | 4-phénylimidazol-1-yl | (CH₂)₄ | H | H | 113-115 |
| 53. | 4-phénylimidazol-1-yl | (CH₂)₄ | H | CH₃ | 109-111 |
| 54. | benzimidazol-1-yl | (CH₂)₄ | H | CH₃ | 114-116 |
| 55. | indol-1-yl | (CH₂)₄ | H | H | 100-102 |
| 56. | indol-1-yl | (CH₂)₄ | H | CH₃ | 64-67 |
| 57. | pyrrolo[2,3-b]pyridin-1-yl | (CH₂)₄ | H | H | 102-104 |
| 58. | pyrrolo[2,3-b]pyridin-1-yl | (CH₂)₄ | H | CH₃ | 52-54 |
| 59. | tétrahydroisoquinolin-2-yl | (CH₂)₄ | H | CH₃ | (320) |
| 60. | 2-oxo-3,4-dihydroquinolin-1(2*H*)-yl | (CH₂)₄ | H | H | (320) |
| 61. | 2-oxo-3,4-dihydroquinolin-1 (*2H*)-yl | (CH₂)₄ | H | CH₃ | (M+H) (334) |
| 62. | pyridin-2-yl | (CH₂)₅ | H | H | 77-79 |
| 63. | pyridin-4-yl | (CH₂)₅ | H | H | 155-157 |
| 64. | pyrimidin-5-yl | (CH₂)₅ | H | H | 115-117 |
| 65. | quinolin-2-yl | (CH₂)₅ | H | H | (316) |
| 66. | quinolin-4-yl | (CH₂)₅ | H | H | 115-117 |
| 67. | isoquinolin-1-yl | (CH₂)₅ | H | H | 134-135 |
| 68. | isoquinolin-4-yl | (CH₂)₅ | H | H | (316) |
| 69. | naphtalèn-1-yl | C≡CCH₂ | H | H | 132-134 |
| 70. | naphtalèn-1-yl | C≡CCH₂ | H | CH₃ | 107-109 |
| 71. | naphtalèn-1-yl | C≡C(CH₂)₃ | H | H | 105-108 |
| 72. | naphtalèn-1-yl | C≡C(CH₂)₃ | H | CH₃ | 73-76 |
| 73. | 4-F-naphtalèn-1-yl | C≡C(CH₂)₃ | H | CH₃ | 96-98 |
| 74. | 3-(pyridin-3-yl)-isoxazol-5-yl | (CH₂)₃ | H | CH₃ | 133-135 |
| 75. | 3-(4-méthoxy-naphtalèn-1-yl)-isoxazol-5-yl | (CH₂)₃ | H | CH₃ | 95-97 |

Les composés de l'invention ont fait l'objet d'essais pharmacologiques permettant de déterminer leur effet inhibiteur de l'enzyme FAAH (Fatty Acid amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimental Therapeutics (1997), 283, 729-734). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est comptée par scintillation liquide.
Dans ces conditions, les composés les plus actifs de l'invention présentent des CI₅₀ (concentration inhibant de 50% l'activité enzymatique contrôle de la FAAH) comprises entre 0,001 et 1 µM.
Par exemple, le composé n° 68 du tableau présente une CI₅₀ de 0,267 µM.

Il apparaît donc que les composés selon l'invention ont une activité inhibitrice sur l'enzyme FAAH.

L'activité *in vivo* des composés de l'invention a été évaluée dans un test d'analgésie.
Ainsi, l'administration intrapéritonéale (i.p.) de PBQ (phénylbenzoquinone, 2 mg/kg dans une solution de chlorure de sodium à 0,9% contenant 5% d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5%, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg. Par exemple, le composé n° 48 du tableau réduit de 53% et de 62% le nombre d'étirements induits par le PBQ, à la dose de 10 mg/kg p.o., respectivement à 60 minutes et à 120 minutes.

L'enzyme FAAH (Chemistry and Physics of Lipids, (2000), 108, 107-121) catalyse l'hydrolyse des dérivés endogènes d'amides et d'esters de différents acides gras tels que la N-arachidonoyléthanolamine (anandamide), la *N*-palmitoyl-éthanolamine, la N-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.
Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoides endogènes et/ ou tous autres substrats métabolisés par l'enzyme FAAH, sont impliqués.
On peut par exemple citer les maladies et les affections suivantes :
La douleur notamment les douleurs aiguës ou chroniques de type neurogène : migraine, douleurs neuropathiques incluant les formes associées au virus de l'herpès et au diabète ;
les douleurs aiguës ou chroniques associées aux maladies inflammatoires : arthrite, arthrite rhumatoïde, ostéoarthrite, spondylite, goutte, vascularite, maladie de Crohn, syndrome du colon irritable ;
les douleurs aiguës ou chroniques périphériques ;
les vertiges, les vomissements, les nausées en particulier celles consécutives à une chimiothérapie ;
les troubles du comportement alimentaire en particulier les anorexies et cachexies de diverses natures ;
les pathologies neurologiques et psychiatriques :
   tremblements, dyskinésies, dystonies, spasticité, comportements compulsifs et obsessionnels, syndrome de Tourette, toutes les formes de dépression et d'anxiété de toute nature et origine, troubles de l'humeur, psychoses ;
   les maladies neuro-dégénératives aiguës et chroniques :
   maladie de Parkinson, maladie d'Alzheimer, démence sénile, chorée de Huntington, lésions liées à l'ischémie cérébrale et aux traumatismes crâniens et médullaires ;
   l'épilepsie ;
   les troubles du sommeil incluant les apnées du sommeil ;
   les maladies cardiovasculaires en particulier hypertension, arythmies cardiaques, artériosclérose, crise cardiaque, ischémies cardiaques ;
   l'ischémie rénale ;
   les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes) ;
   les désordres du système immunitaire, notamment les maladies auto-immunes : psoriasis, lupus érythémateux, maladies du tissu conjonctif ou connectivites, syndrome de Sjögren's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire ;
   les maladies allergiques : l'hypersensibilité immédiate ou retardée, rhinites ou conjonctivites allergiques, dermatites de contact ;
   les maladies infectieuses parasitaires , virales ou bactériennes : SIDA, méningites ; les maladies inflammatoires, notamment les maladies articulaires : arthrite, arthrite rhumatoïde, ostéoarthrite, spondylite, goutte, vascularite, maladie de Crohn, syndrome du colon irritable ; l'ostéoporose ; les affections oculaires : hypertension oculaire, glaucome ;
   les affections pulmonaires : maladies des voies respiratoires, bronchospasmes, toux, asthme, bronchite chronique, obstruction chronique des voies respiratoires, emphysème ;
   les maladies gastro-intestinales: syndrome du colon irritable, désordres inflammatoires intestinaux, ulcères, diarrhées ;
   l'incontinence urinaire et l'inflammation vésicale.

L'utilisation d'un composé de formule (I), à l'état de base, de sel, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223, 75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Les dites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration; le poids et la réponse dudit patient.

## Revendications

1. Composé répondant à la formule générale (I) 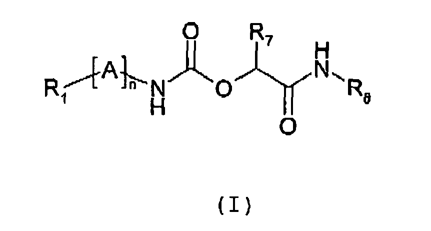 dans laquelle
A est choisi parmi un ou plusieurs groupes X, Y et/ou Z;
X représente un groupe méthylène éventuellement substitué par un ou deux groupes C₁₋₆-alkyle, C₃₋₇)-cycloalkyle ou C₃₋₇-cycloalkyl-C₁₋₃-alkylène ;
Y représente soit un groupe C₂-alcènylène éventuellement substitué par un ou deux groupes C₁₋₆-alkyle, C₃₋₇-cycloalkyle ou C₃₋₇-cycloalkyl-C₁₋₃-alkylène ; soit un groupe C₂-alcynylène ;
Z représente un groupe de formule : 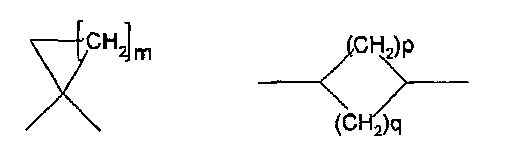 m représente un nombre entier allant de 1 à 5 ;
p et q représentent des nombres entiers et sont définis tels que p+q soit un nombre allant de 1 à 5 ;
n représente un nombre entier allant de 1 à 7 ;
R₁ représente un groupe R₂ éventuellement substitué par un ou plusieurs groupes R₃ et/ou R₄ ;
R₂ représente un groupe choisi parmi un pyridinyle, pyrimidinyle, pyridazinyle, pyrazinyle, triazinyle, pyrrolyle, furanyle, thiènyle, imidazolyle, oxazolyle, thiazolyle, pyrazolyle, isoxazolyle, isothiazolyle, oxadiazolyle, thiadiazolyle, triazolyle, tétrazolyle, naphtalènyle, quinolinyle, tétrahydroquinolinyle, isoquinolinyle, tétrahydroisoquinolinyle, 2-oxo-3,4-dihydroquinolinyle, 1,-oxo-3,4-dihydroisoquinolinyle, quinazolinyle, quinoxalinyle, phthalazinyle, cinnolinyle, naphthyridinyle, benzofuranyle, dihydrobenzofuranyle, benzothiènyle, dihydrobenzothiènyle, indolyle, indolinyle, indazolyle, isoindolyle, benzimidazolyle, benzoxazolyle, benzisoxazolyle, benzothiazolyle, benzisothiazolyle, benzotriazolyle, benzoxadiazolyle, benzothiadiazolyle, pyrrolopyridinyle, furopyridinyle, thiènopyridinyle, imidazopyridinyle, oxazolopyridinyle, thiazolopyridinyle, pyrazolopyridinyle, isoxazolopyridinyle, isothiazolopyridinyle ;
R₃ représente un groupe choisi parmi les atomes d'halogènes, les groupes cyano, nitro, C₁₋₆-alkyle, C₁₋₆-alcoxy, hydroxyle, C₁₋₆-thioalkyle, C₁₋₆-fluoroalkyle, C₁₋₆-fluoroalcoxy, C₁₋₆-fluorothioalkyle, NR₅R₆, NR₅COR₆, NR₅CO₂R₆, NR₅O₂R₆, COR₅, CO₂R₅, CONR₅R₆, SO₂R₅, SO₂NR₅R₆, -O-(C₁₋₃-alkylène)-O;
R₄ représente un groupe choisi parmi les groupes phényle, phényloxy, benzyloxy, naphtalènyle, pyridinyle, pyrimidinyle, pyridazinyle, pyrazinyle ; le ou les groupes R₄ pouvant être substitués par un ou plusieurs groupes R₃ identiques ou différents l'un de l'autre ;
R₅ et R₆ représentent indépendamment l'un de l'autre un atome d'hydxogène ou un groupe C₁₋₆-alkyle, ou forment avec le ou les atomes qui les portent un cycle choisi parmi un cycle azétidine, pyrrolidine, pipéridine, morpholine, thiomorpholine, azépine ou pipérazine, ce cycle étant éventuellement substitué par un groupe C₁₋₆-alkyle ou benzyle; R₇ représente un atome d'hydrogène ou un groupe C₁₋₆-alkyle R₈ représente un atome d'hydrogène ou un groupe C₁₋₆-alkyle, C₃₋₇-cycloalkyle, C₃₋₇-cycloalkyle-C₁₋₃-alkylène;
à l'état de base, de sel d'addition à un acide, d'hydrate ou de solvat.

2. Composé de formule (I) selon la revendication 1,
**caractérisé en ce que** :
A est choisi parmi un ou plusieurs groupes X et/ou Y ;
X représente un groupe méthylène;
Y représente un groupe C₂-alcynylène ;
n représente un nombre entier allant de 1 à 5 ;
R₁ représente un groupe R₂ éventuellement substitué par un ou plusieurs groupes R₃ et/ou R₄ ;
R₂ représente un groupe choisi parmi un pyridinyle, pyrimidinyle, pyridazinyle, imidazolyle, oxazolyle, pyrazolyle, isoxazolyle, oxadiazolyle, naphtalènyle, quinolinyle, isoquinolinyle, dihydroisoquinolinyle, 2-oxo-3,4-dihydroquinolinyle, indolyle, benzimidazolyle, pyrrolopyridinyle ;
R₃ représente un groupe choisi parmi les atomes d'halogènes, les groupes C₁₋₆-alkyle, C₁₋₆-alcoxy, NR₅R₆ ;
R₄ représente un groupe choisi parmi les groupes phényle, naphtalènyle, pyridinyle ; le ou les groupes R₄ pouvant être substitués par un ou plusieurs groupes R₃ identiques ou différents l'un de l'autre ;
R₅ et R₆ représentent indépendamment l'un de l'autre un groupe C₁₋₆-alkyle ;
R₇ représente un atome d'hydrogène ou un groupe C₁₋₆-alkyle ; R₈ représente un atome d'hydrogène ou un groupe C₁₋₆-alkyle, C₃₋₇-cycloalkyle, C₃₋₇-cycloalkyle-C₁₋₃-alkyléne ;
à l'état de base, de sel d'addition à un acide, d'hydrate ou de solvat .

3. Composé de formule (I) selon la revendication 1 ou 2,
**caractérisé en ce que** :
R₂ représente un groupe choisi parmi un pyridinyle, pyrimidinyle, oxazolyle, isoxazolyle, naphtalènyle, quinolinyle, isoquinolinyle.

4. Composé de formule (I) selon l'une quelconque des revendications 1 à 3, **caractérisé en ce que** :
R₇ représente un atome d'hydrogène ;
R₈ représente un atome d'hydrogène ou un groupe C₁₋₆-alkyle ; à l'état de base, de sel d'addition à un acide, d'hydrate ou de solvat .

5. Procédé de préparation d'un composé de formule (I) selon l'une quelconque des revendications 1 à 4, comprenant l'étape consistant à transformer le carbamate-ester de formule générale (IV) 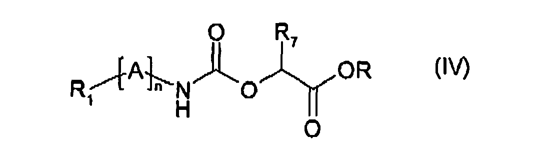 dans laquelle A, n, R₁ et R₇ sont tels que définis dans la formule (I) selon la revendication 1 et R représente un groupe méthyle ou éthyle,
par aminolyse au moyen d'une amine de formule générale R₈NH₂ dans laquelle R₈ est tel que défini dans la formule (I) selon la revendication 1.

6. Procédé de préparation d'un composé de formule (I) selon l'une quelconque des revendications 1 à 4, comprenant l'étape 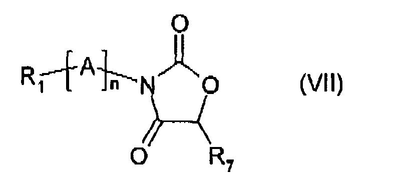 consistant à transformer le dérivé oxazolidine-dione de formule générale (VII)
dans laquelle A, n, R₁ et R₇ sont tels que définis dans la formule (I) selon la revendication 1, par aminolyse au moyen d'une amine de formule générale R₈NH₂
dans laquelle R₈ est tel que défini dans la formule (I) selon la revendication 1.

7. Composé répondant à la formule générale (IV), 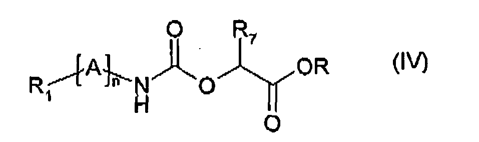 dans laquelle A, n, R₁ et R₁ sont tels que définis dans la formule (I) selon la revendication 1 et R représente un groupe méthyle ou éthyle, les composés suivants étant exclus :
- 2-[({[2-(5-hydroxy-1H-indol-3-yl)ethyl]amino}carbonyl)-oxy]propanoate d'éthyle;
- 2-[({[2-[5-(phenylmethoxy)-1H-indol-3-yl]ethyl}amino)-carbonyl]oxy]propanoate d'éthyle.

8. Composé répondant à la formule générale (VII), 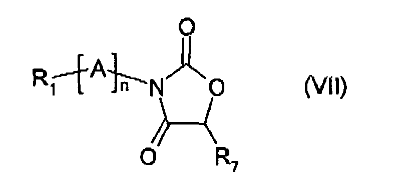 dans laquelle A, n, R₁ et R₇ sont tels que définis dans la formule (I) selon la revendication 1, les composés suivants étant exclus :
- iodure de 2-[2-(2,4-dioxo-3-oxazolidinyl)éthyl]-1-méthylpyridinium ;
- iodure de 2-[2-(2,4-dioxo-3-oxazolidinyl)éthyl]-5-éthyl-1-méthylpyridinium ;
- iodure de 4-[2-(2,4-dioxo-3-oxazolidinyl]éthyl]-1-méthylpyridinium ;
- chlorhydrate de 5-méthyl-3-[2-(4-pyridinyl)éthyl]-2,4-oxazolidinedione;
- chlorhydrate de 5-méthyl-3-[2-(2-pyridinyl)éthyl]-2,4-oxazolidinedione;
- 3-(5-imidazo[1,2-a]pyridin-5-ylpentyl)-2,4-oxazolidinedione;
- 3-[2-(5-méthyl-4-thiazolyl)éthyl]-2,4-oxazolidinedione;
- 3-[2-(1H-pyrrol-2-yl)éthyl]-2,4-oxazolidinedione;
- 3-[2-(2-thiènyl)éthyl]-2,4-oxazolidinedione;
- 3-[3-(2-thiènyl)propyl]-2,4-oxazolidinedione;
- 3-[4-(2-thiènyl)butyl]-2,4-oxazolidinedione;
- 5-méthyl-3-[2-(2-thiènyl)éthyl]-2,4-oxazolidinedione;
- 5-éthyl-3-[2-(2-thiènyl)éthyl]-2,4-oxazolidinedione;
- 3-[2-(3-thiènyl)éthyl]-2,4-oxazolidinedione;
- 3-[2-(5-méthyl-2-thiènyl)éthyl]-2,4-oxazolidinedione;
- 3-[2-(5-acétyl-2-thiènyl)éthyl]-2,4-oxazolidinedione;
- 3-[2-(5-bromo-2-thiènyl)éthyl]-2,4-oxazolidinedione;
- 5-[2-(2,4-dioxo-3-oxazolidinyl)éthyl]-2-thiophènecarboxaldéhyde;
- 3-[3-(1-indolinyl)propyl]-2,4-oxazolidinedione;
- 3-[3-(1-indolinyl)propyl]-5-méthyl-2,4-oxazolidinedione;
- 3-[2-(2-pyridinyl)éthyl]-2,9-oxazolidinedione;
- 3-[2-(5-éthyl-2-pyridinyl)éthyl]-5-méthyl-2,4-oxazolidinedione;
- 5-éthyl-3-[2-(5-éthyl-2-pyridinyl)éthyl]-2,4-oxazolidinedione;
- 3-[2-(5-éthyl-2-pyridinyl)éthyl]-5-isopropyl-2,4-oxazolidinedione;
- 3-[2-(4-pyridinyl)éthyl]-2,4-oxazolidinedione;
- 5-éthyl-3-[2-(4-pyridinyl)éthyl]-2,4-oxazolidinedione:
- 5-éthyl-3-[2-(2-pyridinyl)éthyl]-2,4-oxazolidinedione;
- 5-isopropyl-3-[2-(4-pyridinyl)éthyl]-2,4-oxazolidinedione;
- 5-isopropyl-3-[2-(2-pyridinyl)éthyl]-2,4-oxazolidinedione;
- 3-[2-(5-éthyl-2-pyridinyl)éthyl]-2,4-oxazolidinedione.

9. Composition pharmaceutique contenant au moins un composé de formule (I) selon l'une quelconque des revendications 1 à 4, à l'état de base, de sel, d'hydrate ou de solvat pharmaceutiquement acceptable et éventuellement un ou plusieurs excipients pharmaceutiquement acceptables.

10. Composé de formule (I) selon l'une quelconque des revendications 1 à 4, à l'état de base, de sel, d'hydrate ou de solvat pharmaceutiquement acceptable, pour son utilisation comme médicament.

11. Utilisation d'un composé de formule (I) selon l'une quelconque des revendications 1 à 4, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactérienne, les maladies inflammatoires, l'ostéoporose, les affections oculaires, les affections pulmonaires, les maladies gastro-intestinales ou l'incontinence urinaire.

## Claims

1. Compound corresponding to the general formula (I) 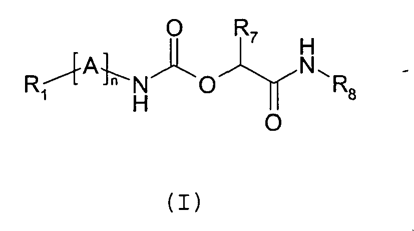 in which
A is chosen from one or more groups X, Y and/or Z;
X represents a methylene group optionally substituted by one or two C₁₋₆-alkyl, C₃₋₇-cycloalkyl or C₃₋₇ cycloalkyl-C₁₋₃-alkylene groups;
Y represents either a C₂-alkenylene group optionally substituted by one or two C₁₋₆-alkyl, C₃₋₇-cycloalkyl or C₃₋₇-cycloalkyl-C₁₋₃-alkylene groups; or a C₂-alkynylene group;
Z represents a group of formula: 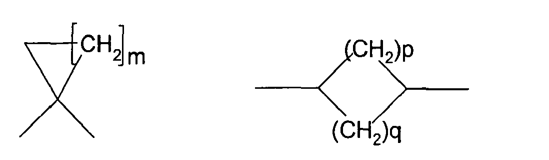 m represents an integer ranging from 1 to 5;
p and q represent integers and are defined such that p+q is a number ranging from 1 to 5;
n represents an integer ranging from 1 to 7;
R₁ represents an R₂ group optionally substituted by one or more R₃ and/or R₄ groups;
R₂ represents a group chosen from a pyridyl, pyrimidinyl, pyridazinyl, pyrazinyl, triazinyl, pyrrolyl, furyl, thienyl, imidazolyl, oxazolyl, thiazolyl, pyrazolyl, isoxazolyl, isothiazolyl, oxadiazolyl, thiadiazolyl, triazolyl, tetrazolyl, naphthyl, quinolinyl, tetrahydroquinolinyl, isoquinolinyl, tetrahydroisoquinolinyl, 2-oxo-3,4-dihydroquinolinyl, 1-oxo-3,4-dihydroisoquinolinyl, quinazolinyl, quinoxalinyl, phthalazinyl, cinnolinyl, naphthyridinyl, benzofuranyl, dihydrobenzofuranyl, benzothienyl, dihydrobenzothienyl, indolyl, indolinyl, indazolyl, isoindolyl, benzimidazolyl, benzoxazolyl, benzisoxazolyl, benzothiazolyl, benzisothiazolyl, benzotriazolyl, benzoxadiazolyl, benzothiadiazolyl, pyrrolopyridyl, furopyridyl, thienopyridyl, imidazopyridyl, oxazolopyridyl, thiazolopyridyl, pyrazolopyridyl, isoxazolopyridyl or isothiazolopyridyl;
R₃ represents a group chosen from halogen atoms or cyano, nitro, C₁₋₆-alkyl, C₁₋₆-alkoxy, hydroxyl, C₁₋₆-thioalkyl, C₁₋₆-fluoroalkyl, C₁₋₆-fluoroalkoxy, C₁₋₆-fluorothioalkyl, NR₅R₆, NR₅COR₆, NR₅CO₂R₆, NR₅SO₂R₆, COR₅, CO₂R₅, CONR₅R₆, SO₂R₅, SO₂NR₅R₆ or -O-(C₁₋₃-alkylene)-O-groups;
R₄ represents a group chosen from phenyl, phenyloxy, benzyloxy, naphthyl, pyridyl, pyrimidinyl, pyridazinyl or pyrazinyl groups; it being possible for the R₄ group or groups to be substituted by one or more R₃ groups which are identical to or different from one another; R₅ and R₆ represent, independently of one another, a hydrogen atom or a C₁₋₆-alkyl group or form, with the atom or atoms which carry them, a ring chosen from an azetidine, pyrrolidine, piperidine, morpholine, thiomorpholine, azepine or piperazine ring, this ring optionally being substituted by a C₁₋₆-alkyl or benzyl group;
R₇ represents a hydrogen atom or a C₁₋₆-alkyl group; R₈ represents a hydrogen atom or a C₁₋₆-alkyl, C₃₋₇-cycloalkyl or C₃₋₇-cycloalkyl-C₁₋₃-alkylene group; in the base form or in the form of an addition salt with an acid, of a hydrate or of a solvate.

2. Compound of formula (I) according to Claim 1,
**characterized in that**:
A is chosen from one or more groups X and/or Y;
X represents a methylene group;
Y represents a C₂-alkynylene group;
n represents an integer ranging from 1 to 5;
R₁ represents an R₂ group optionally substituted by one or more R₃ and/or R₄ groups;
R₂ represents a group chosen from a pyridyl, pyrimidinyl, pyridazinyl, imidazolyl, oxazolyl, pyrazolyl, isoxazolyl, oxadiazolyl, naphthyl, quinolinyl, isoquinolinyl, dihydroisoquinolinyl, 2-oxo-3,4-dihydroquinolinyl, indolyl, benzimidazolyl or pyrrolopyridyl;
R₃ represents a group chosen from halogen atoms or C₁₋₆-alkyl, C₁₋₆-alkoxy or NR₅R₆ groups;
R₄ represents a group chosen from phenyl, naphthyl or pyridyl groups; it being possible for the R₄ group or groups to be substituted by one or more R₃ groups which are identical to or different from one another;
R₅ and R₆ represent, independently of one another, a C₁₋₆-alkyl group;
R₇ represents a hydrogen atom or a C₁₋₆-alkyl group;
R₈ represents a hydrogen atom or a C₁₋₆-alkyl, C₃₋₇-cycloalkyl or C₃₋₇-cycloalkyl-C₁₋₃-alkylene group; in the base form or in the form of an addition salt with an acid, of a hydrate or of a solvate.

3. Compound of formula (I) according to Claim 1 or 2,
**characterized in that**:
R₂ represents a group chosen from a pyridyl, pyrimidinyl, oxazolyl, isoxazolyl, naphthyl, quinolinyl or isoquinolinyl.

4. Compound of formula (I) according to any one of Claims 1 to 3, **characterized in that**:
R₇ represents a hydrogen atom;
R₈ represents a hydrogen atom or a C₁₋₆-alkyl group; in the base form or in the form of an addition salt with an acid, of a hydrate or of a solvate.

5. Process for the preparation of a compound of formula (I) according to any one of Claims 1 to 4, comprising the stage consisting in converting the carbamate ester of general formula (IV) 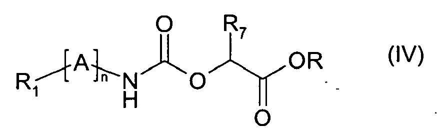 in which A, n, R₁ and R₇ are as defined in the formula (I) according to Claim 1 and R represents a methyl or ethyl group,
by aminolysis using an amine of general formula R₈NH₂, in which R₈ is as defined in the formula (I) according to Claim 1.

6. Process for the preparation of a compound of formula (I) according to any one of Claims 1 to 4, comprising the stage consisting in converting the oxazolidinedione derivative of general formula (VII) 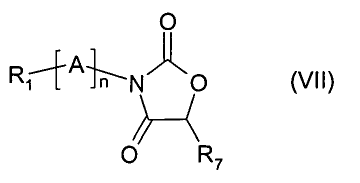 in which A, n, R₁ and R₇ are as defined in the formula (I) according to Claim 1,
by aminolysis using an amine of general formula R₈NH₂, in which R₈ is as defined in the formula (I) according to Claim 1.

7. Compound corresponding to the general formula (IV) , 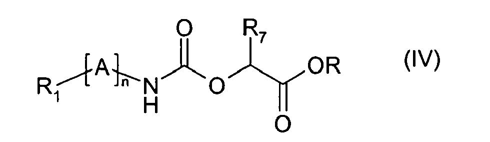 in which A, n, R₁ and R₇ are as defined in the formula (I) according to Claim 1 and R represents a methyl or ethyl group, the following compounds being excluded:
- ethyl 2-[({[2-(5-hydroxy-1H-indol-3-yl)ethyl]amino}carbonyl)oxy]propanoate;
- ethyl 2-[({[2-[[5-(phenylmethoxy)-1H-indol-3-yl] ethyl]amino}carbonyl)oxy]propanoate.

8. Compound corresponding to the general formula (VII) 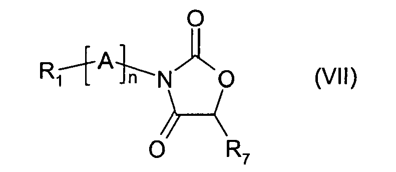 in which A, n, R₁ and R₇ are as defined in the formula (I) according to Claim 1, the following compounds being excluded:
- 2-[2-(2,4-dioxo-3-oxazolidinyl)ethyl]-1-methylpyridinium iodide;
- 2-[2-(2,4-dioxo-3-oxazolidinyl)ethyl]-5-ethyl-1-methylpyridinium iodide;
- 4-[2-(2,4-dioxo-3-oxazolidinyl)ethyl]-1-methylpyridinium iodide;
- 5-methyl-3-[2-(4-pyridyl)ethyl]-2,4-oxazolidinedione hydrochloride;
- 5-methyl-3-[2-(2-pyridyl)ethyl]-2,4-oxazolidinedione hydrochloride;
- 3-[5-(imidazo[1,2-a]pyrid-5-yl)pentyl]-2,4-oxazolidinedione;
- 3-[2-(5-methyl-4-thiazolyl)ethyl]-2,4-oxazolidinedione;
- 3-[2-(1H-pyrrol-2-yl)ethyl]-2,4-oxazolidinedione;
- 3-[2-(2-thienyl)ethyl]-2,4-oxazolidinedione;
- 3-[3-(2-thienyl)propyl]-2,4-oxazolidinedione;
- 3-[4-(2-thienyl)butyl]-2,4-oxazolidinedione;
- 5-methyl-3-[2-(2-thienyl)ethyl]-2,4-oxazolidinedione;
- 5-ethyl-3-[2-(2-thienyl)ethyl]-2,4-oxazolidinedione;
- 3-[2-(3-thienyl)ethyl]-2,4-oxazolidinedione;
- 3-[2-(5-methyl-2-thienyl)ethyl]-2,4-oxazolidinedione;
- 3-[2-(5-acetyl-2-thienyl)ethyl]-2,4-oxazolidinedione;
- 3-[2-(5-bromo-2-thienyl)ethyl]-2,4-oxazolidinedione;
- 5-[2-(2,4-dioxo-3-oxazolidinyl)ethyl]-2-thiophenecarbaldehyde;
- 3-[3-(1-indolinyl)propyl]-2,4-oxazolidinedione;
- 3-[3-(1-indolinyl)propyl]-5-methyl-2,4-oxazolidinedione;
- 3-[2-(2-pyridyl)ethyl]-2,4-oxazolidinedione;
- 3-[2-(5-ethyl-2-pyridyl)ethyl]-5-methyl-2,4-oxazolidinedione;
- 5-ethyl-3-[2-(5-ethyl-2-pyridyl)ethyl]-2,4-oxazolidinedione;
- 3-[2-(5-ethyl-2-pyridyl)ethyl]-5-isopropyl-2,4-oxazolidinedione;
- 3-[2-(4-pyridyl)ethyl]-2,4-oxazolidinedione;
- 5-ethyl-3-[2-(4-pyridyl)ethyl]-2,4-oxazolidinedione;
- 5-ethyl-3-[2-(2-pyridyl)ethyl]-2,4-oxazolidinedione;
- 5-isopropyl-3-[2-(4-pyridyl)ethyl]-2,4-oxazolidinedione;
- 5-isopropyl-3-[2-(2-pyridyl)ethyl]-2,4-oxazolidinedione;
- 3-[2-(5-ethyl-2-pyridyl)ethyl]-2,4-oxazolidinedione.

9. Pharmaceutical composition comprising at least one compound of formula (I) according to any one of Claims 1 to 4, in the base or pharmaceutically acceptable salt, hydrate or solvate form, and optionally one or more pharmaceutically acceptable excipients.

10. Compound of formula (I) according to any one of Claims 1 to 4, in the base or pharmaceutically acceptable salt, hydrate or solvate form, for its use as medicament.

11. Use of a compound of formula (I) according to any one of Claims 1 to 4, in the base or pharmaceutically acceptable salt, hydrate or solvate form, in the preparation of a medicament intended to prevent or treat acute or chronic pain, dizziness, vomiting, nausea, eating disorders, neurological and psychiatric pathologies, acute or chronic neurodegenerative diseases, epilepsy, sleep disorders, cardiovascular diseases, renal ischaemia, cancers, disorders of the immune system, allergic diseases, parasitic, viral or bacterial infectious diseases, inflammatory diseases, osteoporosis, eye conditions, pulmonary conditions, gastrointestinal diseases or urinary incontinence.

## Patentansprüche

1. Verbindung der allgemeinen Formel (I) 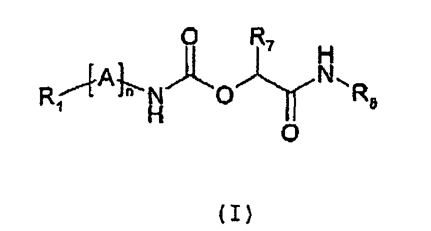 worin
A unter einer oder mehreren Gruppen X, Y und/oder Z ausgewählt ist;
X für eine gegebenenfalls durch eine oder zwei C₁₋₆-Alkyl-, C₃₋₇-Cycloalkyl- oder C₃₋₇-Cycloalkyl-C₁₋₃-alkylengruppen substituierte Methylengruppe steht;
Y entweder für eine gegebenenfalls durch eine oder zwei C₁₋₆-Alkyl-, C₃₋₇-Cycloalkyl- oder C₃₋₇-Cycloalkyl-C₁₋₃-alkylengruppen substituierte C₂-Alkenylengruppe oder eine C₂-Alkinylengruppe steht; Z für eine Gruppe der Formel: 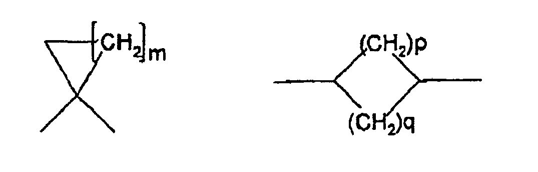 steht;
m für eine ganze Zahl von 1 bis 5 steht;
p und q für ganze Zahlen stehen und so definiert sind, daß p+q für eine ganze Zahl von 1 bis 5 steht;
n für eine ganze Zahl von 1 bis 7 steht;
R₁ für eine gegebenenfalls durch eine oder mehrere Gruppen R₃ und/oder R₄ substituierte Gruppe R₂ steht;
R₂ für eine unter Pyridinyl, Pyrimidinyl, Pyridazinyl, Pyrazinyl, Triazinyl, Pyrrolyl, Furanyl, Thienyl, Imidazolyl, Oxazolyl, Thiazolyl, Pyrazolyl, Isoxazolyl, Isothiazolyl, Oxadiazolyl, Thiadiazolyl, Triazolyl, Tetrazolyl, Naphthalinyl, Chinolinyl, Tetrahydrochinolinyl, Isochinolinyl, Tetrahydroisochinolinyl, 2-Oxo-3,4-dihydrochinolinyl, 1-Oxo-3,4-dihydroisochinolinyl, Chinazolinyl, Chinoxalinyl, Phthalazinyl, Cinnolinyl, Naphthyridinyl, Benzofuranyl, Dihydrobenzofuranyl, Benzothienyl, Dihydrobenzothienyl, Indolyl, Indolinyl, Indazolyl, Isoindolyl, Benzimidazolyl, Benzoxazolyl, Benzisoxazolyl, Benzothiazolyl, Benzisothiazolyl, Benzotriazolyl, Benzoxadiazolyl, Benzothiadiazolyl, Pyrrolopyridinyl, Furopyridinyl, Thienopyridinyl, Imidazopyridinyl, Oxazolopyridinyl, Thiazolopyridinyl, Pyrazolopyridinyl, Isoxazolopyridinyl und Isothiazolopyridinyl ausgewählte Gruppe steht;
R₃ für eine unter Halogenatomen und Cyano-, Nitro-, C₁₋₆-Alkyl-, C₁₋₆-Alkoxy-, Hydroxyl-, C₁₋₆-Thioalkyl-, C₁₋₆-Fluoralkyl-, C₁₋₆-Fluoralkoxy-, C₁-C₆-Fluorthioalkyl-, NR₅R₆-, NR₅COR₆-, NR₅CO₂R₆-, NR₅SO₂R₆-, COR₅-, CO₂R₅-, CONR₅R₆-, SO₂R₅-, SO₂NR₅R₆- und -O-(C₁₋₃-Alkylen)-O-Gruppen ausgewählte Gruppe steht;
R₄ für eine unter Phenyl-, Phenyloxy-, Benzyloxy-, Naphthalinyl-, Pyridinyl-, Pyrimidinyl-, Pyridazinyl- und Pyrazinylgruppen ausgewählte Gruppe steht; wobei die Gruppe bzw. Gruppen R₄ gegebenenfalls durch einen oder mehrere gleiche oder voneinander verschiedene Gruppen R₃ substituiert sein kann bzw. können;
R₅ und R₆ unabhängig voneinander für ein Wasserstoffatom oder eine C₁₋₆-Alkylgruppe stehen oder gemeinsam mit dem Atom bzw. den Atomen, an das bzw. die sie gebunden sind, einen unter einem Azetidin-, Pyrrolidin-, Piperidin-, Morpholin-, Thiomorpholin-, Azepin- oder Piperazinring ausgewählten Ring bilden, welcher gegebenenfalls durch eine C₁₋₆-Alkyl- oder Benzylgruppe substituiert ist;
R₇ für ein Wasserstoffatom oder eine C₁₋₆-Alkylgruppe steht;
R₈ für ein Wasserstoffatom oder eine C₁₋₆-Alkyl-, C₃₋₇-Cycloalkyl- oder C₃₋₇-Cycloalkyl-C₁₋₃alkylengruppe steht;
in Basen-, Säureadditionssalz-, Hydrat- oder Solvatform.

2. Verbindung der Formel (I) nach Anspruch 1, **dadurch gekennzeichnet, daß**:
A unter einer oder mehreren Gruppen X und/oder Y ausgewählt ist;
X für eine Methylengruppe steht;
Y für eine C₂-Alkinylengruppe steht;
n für eine ganze Zahl von 1 bis 5 steht;
R₁ für eine gegebenenfalls durch eine oder mehrere Gruppen R₃ und/oder R₄ substituierte Gruppe R₂ steht;
R₂ für eine unter Pyridinyl, Pyrimidinyl, Pyridazinyl, Imidazolyl, Oxazolyl, Pyrazolyl, Isoxazolyl, Oxadiazolyl, Naphthalinyl, Chinolinyl, Isochinolinyl, Dihydroisochinolinyl, 2-Oxo-3,4-dihydrochinolinyl, Indolyl, Benzimidazolyl und Pyrrolopyridinyl ausgewählte Gruppe steht;
R₃ für eine unter Halogenatomen und C₁₋₆-Alkyl-, C₁₋₆-Alkoxy- und NR₅R₆-Gruppen ausgewählte Gruppe steht;
R₄ für eine unter Phenyl-, Naphthalinyl- und
Pyridinylgruppen ausgewählte Gruppe steht, wobei die Gruppe bzw. Gruppen R₄ gegebenenfalls durch einen oder mehrere gleiche oder voneinander verschiedene Gruppen R₃ substituiert sein kann bzw. können;
R₅ und R₆ unabhängig voneinander für eine C₁₋₆-Alkylgruppe stehen;
R₇ für ein Wasserstoffatom oder eine C₁₋₆-Alkylgruppe steht;
R₈ für ein Wasserstoffatom oder eine C₁₋₆-Alkyl-, C₃₋₇-Cycloalkyl- oder C₃₋₇-Cycloalkyl-C₁₋₃-alkylengruppe steht;
in Basen-, Säureadditionssalz-, Hydrat- oder Solvatform.

3. Verbindung der Formel (I) nach Anspruch 1 oder 2,
**dadurch gekennzeichnet, daß**:
R₂ für eine unter Pyridinyl, Pyrimidinyl, Oxazolyl, Isoxazolyl, Naphthalinyl, Chinolinyl oder Isochinolinyl ausgewählte Gruppe steht.

4. Verbindung der Formel (I) nach einem der Ansprüche 1 bis 3, **dadurch gekennzeichnet, daß**
R₇ für ein Wasserstoffatom steht;
R₈ für ein Wasserstoffatom oder eine C₁₋₆-Alkylgruppe steht;
in Basen-, Säureadditionssalz-, Hydrat- oder Solvatform.

5. Verfahren zur Herstellung einer Verbindung der Formel (I) nach einem der Ansprüche 1 bis 4, bei dem man den Carbamatester der allgemeinen Formel (IV) 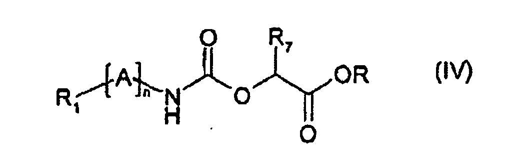 worin A, n, R₁ und R₇ die in der Formel (I) nach Anspruch 1 angegebene Bedeutung besitzen und R für eine Methyl- oder Ethylgruppe steht,
durch Aminolyse mit einem Amin der allgemeinen Formel R₈NH₂, worin R₈ die in der Formel (I) nach Anspruch 1 angegebene Bedeutung besitzt, umwandelt.

6. Verfahren zur Herstellung einer Verbindung der Formel (I) nach einem der Ansprüche 1 bis 4, bei dem man das Oxazolidindionderivat der allgemeinen Formel (VII) 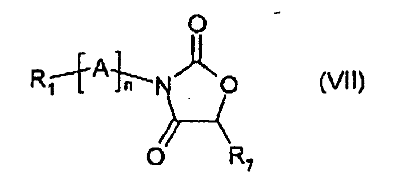 worin A, n, R₁ und R₇ die in der Formel (I) nach Anspruch 1 angegebene Bedeutung besitzen,
durch Aminolyse mit einem Amin der allgemeinen Formel R₈NH₂, worin R₈ die in der Formel (I) nach Anspruch 1 angegebene Bedeutung besitzt, umwandelt.

7. Verbindung der allgemeinen Formel (IV) 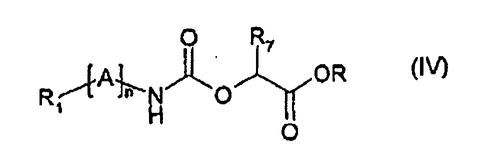 worin A, n, R₁ und R₇ die in der Formel (I) nach Anspruch 1 angegebene Bedeutung besitzen und R für eine Methyl- oder Ethylgruppe steht, wobei die folgenden Verbindungen ausgeschlossen sind:
- 2-[({[2-(5-Hydroxy-1H-indol-3-yl)ethyl]amino}carbonyl)oxy]propansäureethylester;
- 2-[({[2-[5-(Phenylmethoxy)-1H-indol-3-yl]-ethyl]amino}carbonyl)oxy]propansäureethylester.

8. Verbindung der allgemeinen Formel (VII) 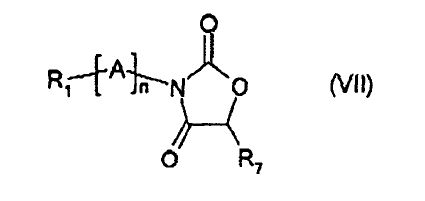 worin A, n, R₁ und R₇ die in der Formel (I) nach Anspruch 1 angegebene Bedeutung besitzen, wobei die folgenden Verbindungen ausgeschlossen sind:
- 2-[2-(2,4-Dioxo-3-oxazolidinyl)ethyl]-1-methylpyridiniumiodid;
- 2-[2-(2,4-Dioxo-3-oxazolidinyl)ethyl]-5-ethyl-1-methylpyridiniumiodid;
- 4-[2-(2,4-Dioxo-3-oxazolidinyl)ethyl]-1-methylpyridiniumiodid;
- 5-Methyl-3-[2-(4-pyridinyl)ethyl]-2,4-oxazolidindion-hydrochlorid;
- 5-Methyl-3-[2-(2-pyridinyl)ethyl]-2,4-oxazolidindion-hydrochlorid,
- 3-[5-(Imidazo[1,2-a]pyridin-5-yl)pentyl]-2,4-oxazolidindion;
- 3-[2-(5-Methyl-4-thiazolyl)ethyl]-2,4-oxazolidindion;
- 3-[2-(1H-Pyrrol-2-yl)ethyl]-2,4-oxazolidindion;
- 3-[2-(2-Thienyl)ethyl]-2,4-oxazolidindion;
- 3-[3-(2-Thienyl)propyl]-2,4-oxazolidindion;
- 3-[4-(2-Thienyl)butyl]-2,4-oxazolidindion;
- 5-Methyl-3-[2-(2-thienyl)ethyl]-2,4-oxazolidindion;
- 5-Ethyl-3-[2-(2-thienyl)ethyl]-2,4-oxazolidindion;
- 3-[2-(3-Thienyl)ethyl]-2,4-oxazolidindion;
- 3-[2-(5-Methyl-2-thienyl)ethyl]-2,4-oxazolidindion;
- 3-[2-(5-Acetyl-2-thienyl)ethyl]-2,4-oxazolidindion;
- 3-[2-(5-Brom-2-thienyl)ethyl]-2,4-oxazolidindion;
- 5-[2-(2,4-Dioxo-3-oxazolidinyl)ethyl]-2-thiophencarbaldehyd;
- 3-[3-(1-Indolinyl)propyl]-2,4-oxazolidindion;
- 3-[3-(1-Indolinyl)propyl]-5-methyl-2,4-oxazolidindion;
- 3-[2-(2-Pyridinyl)ethyl]-2,4-oxazolidindion;
- 3-[2-(5-Ethyl-2-pyridinyl)ethyl]-5-methyl-2,4-oxazolidindion;
- 5-Ethyl-3-[2-(5-ethyl-2-pyridinyl)ethyl]-2,4-oxazolidindion;
- 3-[2-(5-Ethyl-2-pyridinyl)ethyl]-5-isopropyl-2,4-oxazolidindion;
- 3-[2-(4-Pyridinyl)ethyl]-2,4-oxazolidindion;
- 5-Ethyl-3-[2-(4-pyridinyl)ethyl]-2,4-oxazolidindion;
- 5-Ethyl-3-[2-(2-pyridinyl)ethyl]-2,4-oxazolidindion;
- 5-Isopropyl-3-[2-(4-pyridinyl)ethyl]-2,4-oxazolidindion;
- 5-Isopropyl-3-[2-(2-pyridinyl)ethyl]-2,4-oxazolidindion;
- 3-[2-(5-Ethyl-2-pyridinyl)ethyl]-2,4-oxazolidindion.

9. Pharmazeutische Zusammensetzung, enthaltend mindestens eine Verbindung der Formel (I) nach einem der Ansprüche 1 bis 4 in pharmazeutisch unbedenklicher Basen-, Salz-, Hydrat- oder Solvatform und gegebenenfalls einen oder mehrere pharmazeutisch unbedenkliche Hilfsstoffe.

10. Verbindung der Formel (I) nach einem der Ansprüche 1 bis 4 in pharmazeutisch unbedenklicher Basen-, Salz-, Hydrat- oder Solvatform zur Verwendung als Arzneimittel.

11. Verwendung einer Verbindung der Formel (I) nach einem der Ansprüche 1 bis 4 in pharmazeutisch unbedenklicher Basen-, Salz-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten und chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-Darm-Erkrankungen oder Harninkontinenz.
